# EUROPEAN PATENT SPECIFICATION

(11) **EP 3 793 586 B1**
(45) Date of publication and mention of the grant of the patent: **10.04.2024**
(21) Application number: 19803150.2
(22) Date of filing: 15.05.2019
(51) Int. Cl.: A61K 38/55, A61K 38/17, C07K 14/705, C07K 19/00, A61P 37/06

(54) **SOLUBLE COMPLEMENT RECEPTOR TYPE 1 VARIANTS AND USES THEREOF**
LÖSLICHE KOMPLEMENT-REZEPTOR-TYP-1-VARIANTEN UND DEREN VERWENDUNGEN
VARIANTS DE TYPE 1 DU RÉCEPTEUR DU COMPLÉMENT SOLUBLE ET UTILISATIONS ASSOCIÉES

(30) Priority: 16.05.2018 AU 2018901703
(43) Date of publication of application: 24.03.2021
(60) Divisional application: 24159300.3
(73) Proprietor: CSL Limited, Melbourne, VIC 3000 (AU)
(72) Inventor: HARDY, Matthew, Doreen, Victoria 3754 (AU); ROWE, Tony, Beaumaris, Victoria 3193 (AU); CAO, Zhihui (Helen), Doncaster East, Victoria 3109 (AU); BAZ MORELLI, Adriana, Carlton, Victoria 3053 (AU); WYMANN, Sandra, 3110 Muensingen (CH)
(74) Representative: CSL Global IP
(86) International application number: PCT/AU2019/050456
(87) International publication number: WO 2019/218009

(56) References cited:
- WO-A1-91/05047
- WO-A1-94/26786
- WO-A1-2011/143637
- WO-A2-2012/058479
- US-A- 5 981 481
- Ghiran Ionita ET AL: "CR1" In: "The Complement FactsBook", 1 January 2018 (2018-01-01), Elsevier, US, XP055894682, ISBN: 978-0-12-810420-0 pages 295-308, DOI: 10.1016/B978-0-12-810420-0.00028-6, Retrieved from the Internet: URL:http://dx.doi.org/10.1016/B978-0-12-81 0420-0.00028-6> * the whole document *
- WYMANN SANDRA ET AL: "A novel soluble complement receptor 1 fragment with enhanced therapeutic potential", JOURNAL OF BIOLOGICAL CHEMISTRY, vol. 296, 1 January 2021 (2021-01-01), page 100200, XP055894876, US ISSN: 0021-9258, DOI: 10.1074/jbc.RA120.016127
- THEROUX, P et al.: "Complement activity and pharmacological inhibition in cardiovascular disease", Canadian Journal of Cardiology, vol. 22 , XP008070769,
- SCESNEY, S.M. et al.: "A soluble deletion mutant of the human complement receptor type 1, which lacks the C4b binding site, is a selective inhibitor of the alternative complement pathway", European Journal of Immunology, vol. 26, 1996, pages 1729-1735, XP001147715, DOI: 10.1002/eji.1830260810
- ASGHAR, S.S. et al.: "Therapeutic Inhibiton of the Complement System. Y2K Update", Frontiers in Bioscience, vol. 5, 2000, pages e63-82, XP001055180,
- GILLINOV, A.M. et al.: "Complement Inhibition With Soluble Complement Receptor Type 1 in Cardiopulmonary Bypass", Annals of Thoracic Surgery, vol. 55, 1993, pages 619-624, XP001085121,
- KRYCH-GOLDBERG, M. et al.: "Decay Accelerating Activity of Complement Receptor Type 1 ( CD 35", The Journal of Biological Chemistry, vol. 274, no. 44, 1999, pages 31160-31168, XP055653587,

## Description

### RELATED APPLICATION DATA

The present application claims priority from Australian Patent Application No. 2018901703 entitled "Soluble complement receptor type I variants and uses thereof' filed on 16 May 2018.

### SEQUENCE LISTING

The present application is filed with a Sequence Listing in electronic form.

### FIELD

The present disclosure relates to soluble complement receptor type 1 variants and uses thereof.

### BACKGROUND

The complement system is part of the innate immune system and is comprised of a number of cell-surface and soluble proteins that play a role in elimination of foreign microorganisms, whilst protecting the host from complement-related damage.

The complement system comprises soluble components C1-C9 and becomes activated when its primary components are fragmented and the fragments, alone or with other proteins, activate additional complement proteins resulting in a proteolytic cascade. Activation of the complement system leads to increased vascular permeability, chemotaxis of phagocytic cells, activation of inflammatory cells, opsonization of foreign particles, direct killing of cells and tissue damage.

The three pathways of the complement system all eventually lead to the formation of a membrane attack complex (MAC) comprising complement components C5b, C6, C7, C8 and C9, as well as the release of the anaphylotoxins C3a and C5a. However, each pathway is triggered differently. The classical pathway is triggered in response to antigen-antibody complexes and involves activation of complement component C4 by complement component C1s leading to sequential activation of complement components C2, C3 and C5 and formation of a MAC. The lectin pathway involves activation mannan-binding lectin serine protease 1 (MASP1) and MASP2 by binding of mannose-binding lectin (MBL) to respective carbohydrates on the surface of pathogens. Activated MASP1/MASP2 activates C4 leading to sequential activation of complement components C2, C3 and C5 and formation of a MAC. Unlike the classical and lectin pathways, the alternative pathway does not involve activation of C4 and C2, but is triggered by pathogen surfaces causing activation of C3 via Factor B, Factor D and Properdin, followed by activation of C5 and the formation of a MAC. C3 and C5 can also be activated by proteins of the coagulation cascade.

Complement receptor type 1 (CR1) is a principal regulator of the activation of complement. CR1 (also known as C3b/C4b receptor) is a membrane-bound protein present on erythrocytes, macrophages/monocytes, granulocytes, B cells, some T cells, splenic follicular dendritic cells and glomerular podocytes. A minor amount of soluble CR1 (sCR1) is cleaved from the cell surface CR1; a recombinant version of this soluble molecule has previously been generated and is known as TP10. CR1 is a negative regulator of C3 activation and thus sCR1 can inhibit each of the classical, lectin and alternative pathways.

sCR1 has a relatively short half-life of approximately 70 hours (3 days) (Zimmerman et al., 2000 Crit Care Med 28: 3149-3154). sCR1 variants having one or more amino acid substitutions and/or sCR1 truncation variants that retain complement inhibitory activity have been previously described (e.g., WO1994000571). For example, WO 2012/058479 relates to a method for inhibiting complement activation in brain-dead organ kidney donors and various sCR1 variants are disclosed, including a variant lacking LHR-A. WO 94/26786 relates to a composition comprising a soluble complement regulatory protein moiety which has a short consensus repeat structural motif and which binds a complement component, linked to a carbohydrate moiety that is a selectin ligand and some embodiments comprise sCR1 variants lacking LHR-A. This document also mentions in Example 3 a sCR1[des-D] variant. P. Theroux et al. (Can J Cardiol. 2006;22 Suppl B:18B-24B), S. M. Scesney (Eur J Immunol. 1996; 26(8): 1729-35) and S. S. Asghar (Front Biosci. 2000; 5:E63-81) disclose further information on sCR1 variants lacking LHR-A.

However, it will be clear to the skilled person that there is an on-going need in the art for sCR1 variants with improved activity, such as increased complement inhibitory activity, and/or increased half-life.

### SUMMARY

The present disclosure is based on the inventors' identification of soluble complement receptor type 1 (sCR1) variants with increased inhibitory complement activity in a subject.

In producing the present disclosure, the inventors produced sCR1 truncation variants comprising defined amino acid sequences corresponding to one or more long homologous repeat (LHR) regions (i.e., LHR-A, LHR-B, LHR-C and/or LHR-D). The inventors studied the effects of each sCR1 variant for complement inhibiting activity. sCR1 truncation variants of the present disclosure have improved or increased inhibitory activity in all three complement pathways. The inventors have determined that sCR1 variants comprising residues 42 to 1392 of SEQ ID NO: 1 and lacking LHR-D have increased inhibitory activity compared to another form of sCR1.

The findings by the inventors provide the basis for soluble complement receptor type 1 (sCR1) variants for use in methods of inhibiting complement activity in a subject, comprising administering a sCR1 variant to the subject. The findings by the inventors also provide the basis for soluble complement receptor type 1 (sCR1) variants for use in methods for treating or preventing a disorder, e.g., a complement mediated disorder, in a subject.

The present disclosure provides, a soluble complement receptor type 1 (sCR1) variant for use in a method of inhibiting complement activity in a subject, the method comprising administering a soluble complement receptor type 1 (sCR1) variant to the subject, the sCR1 variant comprising an amino acid sequence corresponding to amino acids 42 to 1392 of SEQ ID NO: 1, wherein the sCR1 variant comprises long homologous repeat (LHR) regions LHR-A, LHR-B and LHR-C, but is lacking LHR-D.

The sCR1 variant comprises: an amino acid sequence corresponding to amino acids 42 to 1392 of SEQ ID NO: 1 (e.g., lacking amino acid residues 1393 to 1971 of SEQ ID NO: 1). In one example, the sCR1 variant consists of an amino acid sequence corresponding to amino acids 42 to 1392 of SEQ ID NO: 1. In one example, the sCR1 variant consists of an amino acid sequence corresponding to amino acids 42 to 1392 of SEQ ID NO: 1 or comprises an amino acid sequence corresponding to amino acids 42 to 1392 of SEQ ID NO: 1 (e.g., lacking amino acid residues 1393 to 1971 of SEQ ID NO: 1).

The inventors have shown that such a sCR1 variant has improved complement inhibitory activity compared to a sCR1 variant comprising amino acids 42 to 1971 of SEQ ID NO: 1. This finding was unexpected since the region of CR1 in amino acids 1393 to 1971 binds to C1q and mannose binding lectin (MBL), and its removal might reasonably have been expected to be deleterious to complement inhibitory activity or to have no effect.

In one example, the sCR1 variant does not consist or comprise an amino acid sequence corresponding to amino acids 1 to 1971 of SEQ ID NO: 1. In one example, the sCR1 variant does not consist or comprise an amino acid sequence corresponding to amino acids 42 to 1971 of SEQ ID NO: 1.

In one example, the sCR1 variant of the present disclosure optionally comprises one or more amino acid substitutions, deletions or insertions of any sequence disclosed herein. Amino acid substitutions suitable for use in the present disclosure will be apparent to the skilled person and include naturally-occurring substitutions and engineered substitutions.

In one example, a sCR1 variant of the present disclosure comprises one or more conservative amino acid substitutions compared to a sequence disclosed herein. In some examples, the sCR1 variant comprises 10 or fewer, e.g., 9 or 8 or 7 or 6 or 5 or 4 or 3 or 2 or 1 conservative amino acid substitutions.

In one example, a sCR1 variant of the present disclosure comprises one or more non-conservative amino acid changes. For example, non-conservative amino acid substitutions increase half-life, reduce immunogenicity, and/or increase inhibitory activity of a sCR1 variant of the present disclosure. In one example, the sCR1 variant comprises fewer than 6 or 5 or 4 or 3 or 2 or 1 non-conservative amino acid substitutions.

In one example, a sCR1 variant of the present disclosure comprises a sequence at least about 85% or about 90% or about 95% or about 97% or about 98% or about 99% identical to a sequence disclosed herein.

In one example, the sCR1 variant comprises an amino acid sequence at least about 85% or about 90% or about 95% or about 97% or about 98% or about 99% identical to an amino acid sequence corresponding to amino acids 42 to 1392 of SEQ ID NO: 1 (e.g., lacking amino acid residues 1393 to 1971 of SEQ ID NO: 1). For example, the sCR1 variant comprises an amino acid sequence about 85% identical to an amino acid sequence corresponding to amino acids 42 to 1392 of SEQ ID NO: 1. In another example, the sCR1 variant comprises an amino acid sequence about 90% identical to an amino acid sequence corresponding to amino acids 42 to 1392 of SEQ ID NO: 1. In another example, the sCR1 variant comprises an amino acid sequence about 95% identical to an amino acid sequence corresponding to amino acids 42 to 1392 of SEQ ID NO: 1. In a further example, the sCR1 variant comprises an amino acid sequence about 97% identical to an amino acid sequence corresponding to amino acids 42 to 1392 of SEQ ID NO: 1. In one example, the sCR1 variant comprises an amino acid sequence about 98% identical to an amino acid sequence corresponding to amino acids 42 to 1392 of SEQ ID NO: 1. In another example, the sCR1 variant comprises an amino acid sequence about 99% identical to an amino acid sequence corresponding to amino acids 42 to 1392 of SEQ ID NO: 1.

In one example, the sCR1 variant of the present disclosure has increased inhibitory activity compared to a sCR1 comprising a sequence set forth in SEQ ID NO: 2. For example, the complement inhibitory activity of the sCR1 variant of the present disclosure is increased by at least about 1.5 fold, or about 2 fold, or about 3 fold, or about 3.5 fold, or about 4 fold, or about 5 fold, or about 6 fold, or about 8 fold, or about 10 fold compared to a sCR1 comprising a sequence set forth in SEQ ID NO: 2.

Methods for determining the inhibitory activity of the sCR1 variant will be apparent to the skilled person and/or described herein. In one example, complement inhibitory activity is determined using an *in vitro* assay. For example, complement activity is measured using an enzyme immunoassay (e.g., an immunoassay that measures complement activation, such as a Wieslab^{®} complement assay kit). For example, complement inhibitory activity is determined using labelled antibodies specific for an antigen or an epitope produced during complement activation (e.g., C5b-9 or an epitope present in C5b-C9). In one example, the wells of a microtitre plate are coated with specific activators of the classical, lectin or alternative pathway. In one example, the sCR1 variant is incubated with normal human serum and appropriate assay diluent (i.e., a diluent comprising appropriate blocking components to ensure specific activation of the classical, lectin or alternative pathway) and added to microtitre plate wells coated with specific activators of the classical, lectin or alternative pathway and the amount of C5b-9 complex formed is detected using a specific alkaline phosphatase labelled antibody to the C5b-9. In one example, the amount of complement activation product (i.e., C5b-9) produced is proportional to the functional activity of the complement pathway. In one example, the half maximal inhibitor concentration (i.e., IC₅₀) is determined. For example, the IC₅₀ of the sCR1 variant is determined and compared to the IC₅₀ of a sCR1 comprising a sequence set forth in SEQ ID NO: 2. In another example, complement inhibitory activity is determined using a hemolysis assay (e.g., classical pathway (i.e., CH50) and alternative pathway (ApH50) inhibition assays).

In one example, the sCR1 variant has increased inhibitory activity in the classical pathway, the lectin pathway and/or alternative complement pathway compared to a sCR1 comprising a sequence set forth in SEQ ID NO: 2.

In one example, the sCR1 variant has increased inhibitory activity in the classical complement pathway compared to a sCR1 comprising a sequence set forth in SEQ ID NO: 2. For example, the inhibitory activity of the sCR1 variant of the present disclosure in the classical complement pathway is increased by at least 1.25 fold, or about 1.5 fold, or about 1.75 fold, or about 2 fold, or about 2.5 fold, or about 3 fold, or about 3.5 fold, or about 4 fold, or about 5 fold compared to a sCR1 comprising a sequence set forth in SEQ ID NO: 2.

In one example, the sCR1 variant of the present disclosure has an IC₅₀ in a classical complement assay (e.g., Wieslab complement assay) that is less than a sCR1 comprising a sequence set forth in SEQ ID NO: 2. For example, the sCR1 variant of the present disclosure has an IC₅₀ in a classical complement assay (e.g., Wieslab complement assay) of less than about 1.0nM, such as about 0.95nM, or about 0.90nM, or about 0.85nM, or about 0.80nM, or about 0.75nM, or about 0.70nM. In one example, the sCR1 variant of the present disclosure has an IC₅₀ in a classical complement assay (e.g., Wieslab complement assay) of between about 0.85nM and 0.90nM, such as about 0.88nM. In one example, the sCR1 variant of the present disclosure has an IC₅₀ in a classical complement assay (e.g., Wieslab complement assay) of less than about 0.65nM, or about 0.60nM, or about 0.55nM, or about 0.50nM, or about 0.45nM, or about 0.40nM, or about 0.35nM, or about 0.30nM, or about 0.25nM, or about 0.20nM, or about 0.15nM, or about 0.10nM. In one example, the sCR1 variant of the present disclosure has an IC₅₀ in a classical complement assay (e.g., Wieslab complement assay) of between about 0.35nM and 0.45nM, such as about 0.40nM.

In one example, the sCR1 variant has increased inhibitory activity in the lectin complement pathway compared to a sCR1 comprising a sequence set forth in SEQ ID NO: 2. For example, the inhibitory activity of the sCR1 variant of the present disclosure in the lectin complement pathway is increased by at least 1.25 fold, or about 1.5 fold, or about 1.75 fold, or about 2 fold, or about 2.5 fold, or about 3 fold, or about 3.5 fold, or about 4 fold, or about 5 fold compared to a sCR1 comprising a sequence set forth in SEQ ID NO: 2.

In one example, the sCR1 variant of the present disclosure has an IC₅₀ in a lectin complement assay (e.g., Wieslab complement assay) that is less than a sCR1 comprising a sequence set forth in SEQ ID NO: 2. For example, the sCR1 variant of the present disclosure has an IC₅₀ in a lectin complement assay (e.g., Wieslab complement assay) of less than about 0.60nM, or about 0.55nM, or about 0.50nM. In one example, the sCR1 variant of the present disclosure has an IC₅₀ in a lectin complement assay (e.g., Wieslab complement assay) of between about 0.50nM and 0.60nM, such as about 0.547nM. In one example, the sCR1 variant of the present disclosure has an IC₅₀ in a lectin complement assay (e.g., Wieslab complement assay) of less than about 0.50nM, or about 0.45nM, or about 0.40nM, or about 0.35nM, or about 0.30nM. In one example, the sCR1 variant of the present disclosure has an IC₅₀ in a lectin complement assay (e.g., Wieslab complement assay) of between about 0.40nM and 0.45nM, such as about 0.43nM.

In one example, the sCR1 variant has increased inhibitory activity in the alternative complement pathway compared to a sCR1 comprising a sequence set forth in SEQ ID NO: 2. For example, the inhibitory activity of the sCR1 variant of the present disclosure in the alternative complement pathway is increased by at least 1.25 fold, or about 1.5 fold, or about 1.75 fold, or about 2 fold, or about 2.5 fold, or about 3 fold, or about 3.5 fold, or about 4 fold, or about 5 fold compared to a sCR1 comprising a sequence set forth in SEQ ID NO: 2.

In one example, the sCR1 variant of the present disclosure has an IC₅₀ in an alternative complement assay (e.g., Wieslab complement assay) that is less than a sCR1 comprising a sequence set forth in SEQ ID NO: 2. For example, the sCR1 variant of the present disclosure has an IC₅₀ in an alternative complement assay (e.g., Wieslab complement assay) of less than about 0.75nM, or about 0.70nM, or about 0.65nM, or about 0.60nM, or about 0.55nM, or about 0.50nM, or about 0.45nM, or about 0.40nM, or about 0.35nM, or about 0.30nM, or about 0.25nM. In one example, the sCR1 variant of the present disclosure has an IC₅₀ in an alternative complement assay (e.g., Wieslab complement assay) of between about 0.35nM and about 0.40nM, such as about 0.38nM. In one example, the sCR1 variant of the present disclosure has an IC₅₀ in an alternative complement assay (e.g., Wieslab complement assay) of between about 0.25nM and about 0.30nM, such as about 0.27nM.

The sCR1 variant of the present disclosure comprises LHR regions consisting of LHR-A, LHR-B and LHR-C, but lacking LHR-D.

In one example, LHR region LHR-A comprises an amino acid sequence corresponding to amino acids 42 to 489 of SEQ ID NO: 1. For example, the LHR-A region comprises an amino acid sequence set forth in SEQ ID NO: 13. In one example, LHR region LHR-A comprises short consensus repeat (SCR) sequences 1 to 7. For example, SCR sequences 1 to 3 (i.e., Site 1) are capable of binding to C4b.

In one example, LHR region LHR-B comprises an amino acid sequence corresponding to amino acids 490 to 939 of SEQ ID NO: 1. For example, the LHR-B region comprises an amino acid sequence set forth in SEQ ID NO: 14. In one example, LHR region LHR-B comprises SCR sequences 8 to 14. For example, SCR sequences 8 to 10 (i.e., Site 2) are capable of binding to C3b and C4b.

In one example, LHR region LHR-C comprises an amino acid sequence corresponding to amino acids 940 to 1392 of SEQ ID NO: 1. For example, the LHR-C region comprises an amino acid sequence set forth in SEQ ID NO: 15. In one example, LHR region LHR-C comprises SCR sequences 15 to 21. For example, SCR sequences 15 to 17 are capable of binding to C3b and C4b.

In one example, LHR region LHR-D comprises an amino acid sequence corresponding to amino acids 1393 to 1971 of SEQ ID NO: 1. For example, the LHR-D region comprises an amino acid sequence set forth in SEQ ID NO: 16. In one example, LHR region LHR-D comprises SCR sequences 22 to 28. For example, SCR sequences 22 to 28 are capable of binding to C1q and MBL.

In one example, the sCR1 variant of the present disclosure comprises SCR sequences SCR-1 to SCR-21 (e.g., lacking SCR-22 to SCR-28).

In one example, the sCR1 variant is monomeric (i.e., one copy of the sCR1 variant).

In one example, the sCR1 variant is dimeric, or dimerized (i.e., two copies of a sCR1 variant are linked in a fusion protein).

In one example, the sCR1 variant is multimeric, or multimerized (i.e., multiple copies of a sCR1 variant are linked in a fusion protein).

In one example, two or more of the same sCR1 variant are fused (i.e., expressed as a fusion protein).

In one example, two or more different sCR1 variants are fused (i.e., expressed as a fusion protein).

In one example, the dimerized or multimerized sCR1 variant comprises a linker between the sCR1 variants.

In one example, the disclosure provides a multimeric protein comprising two or more sCR1 variants comprising a multimerization domain, wherein the multimerization domains interact to form the multimeric protein.

In one example, each sCR1 variant in the multimeric protein comprises one sCR1 variant. In another example, one or more sCR1 variants in the multimeric protein comprises two or more sCR1 variants, e.g., the sCR1 variants are linked in a fusion protein.

In one example, the multimerization domain comprises an immunoglobulin hinge domain.

In one example, the multimerization domain is a leucine zipper domain, a cystine knot or an antibody Fc region.

In one example, the multimerized sCR1 variant is linear.

In one example, the multimerized sCR1 variant is circular.

The present disclosure provides a sCR1 variant as described herein in any example (e.g., the description of sCR1 variants in relation to a method for inhibiting complement activity shall be taken to apply to the following description in relation to sCR1 variants *per se*) conjugated to a half-life extending moiety or a further soluble complement inhibitor. In one example, the sCR1 variant is chemically conjugated to the half-life extending moiety or a further soluble complement inhibitor. In another example, the sCR1 variant is fused, e.g., expressed as a fusion protein, with the half-life extending moiety or a further soluble complement inhibitor. In one example, the half-life extending moiety or a further soluble complement inhibitor is conjugated to the C-terminus of the sCR1 variant. In one example, the half-life extending moiety or a further soluble complement inhibitor is conjugated to the N-terminus of the sCR1 variant.

In one example, the sCR1 variant of the present disclosure is conjugated to a half-life extending moiety. For example, the half-life extending moiety is selected from the group consisting of albumin or functional fragments or variants thereof, human serum albumin or functional fragments or variants thereof, immunoglobulins or functional fragments thereof, afamin, alpha-fetoprotein, vitamin D binding protein, and polymers.

In one example, the immunoglobulin or functional fragment thereof is an antibody fragment that binds to albumin. For example, the half-life extending moiety is an antibody Fc region (i.e., a monomeric or dimeric immunoglobulin Fc region), e.g., a human IgG₁ Fc region or a human IgG₄ Fc region or a stabilized human IgG₄ Fc region. For example, the Fc region is a human IgG₄ Fc region. In one example, the antibody Fc region is modified to prevent dimerization, (e.g., as discussed herein). For example, the antibody Fc region is a monomeric Fc region. In one example, the Fc fragment and/or variant thereof, comprises one or more amino acid substitutions, deletions or insertions. Amino acid substitutions suitable for use in the present disclosure will be apparent to the skilled person and include naturally-occurring substitutions and engineered substitutions such as those described, for example, in WO2000042072, WO2002060919, WO2004035752 and WO2006053301.

In one example, the sCR1 variant is fused to an antibody Fc region at its C-terminus. For example, the sCR1 variant consists of an amino acid sequence corresponding to amino acids 42 to 1392 of SEQ ID NO: 1 or comprises an amino acid sequence corresponding to amino acids 42 to 1392 of SEQ ID NO: 1 (e.g., lacking amino acid residues 1393 to 1971 of SEQ ID NO: 1) and is fused to an antibody Fc region at its C-terminus. The inventors found that such a fusion protein had greater than expected complement inhibitory activity compared to the protein lacking the Fc region. Given that the Fc region dimerizes, an increase in activity of about two-fold would be expected, however the inventors observed up to about eight-fold improvement.

In one example, the conjugated sCR1 variant of the present disclosure has a longer serum half-life compared to a sCR1 variant conjugate comprising a sCR1 set forth in SEQ ID NO: 2. Examples of increased serum half-life and assays for determining serum half-life are described herein and are to be taken to apply *mutatis mutandis* to this example of the disclosure.

In one example, the half-life extending moiety is albumin, a functional fragment or variant thereof. In one example, the albumin, functional fragment or variant thereof is serum albumin, such as human serum albumin. In one example, the albumin, functional fragment or variant thereof, comprises one or more amino acid substitutions, deletions or insertions, e.g., no more than 5 or 4 or 3 or 2 or 1 substitutions. Amino acid substitutions suitable for use in the present disclosure will be apparent to the skilled person and include naturally-occurring substitutions and engineered substitutions such as those described, for example, in WO2011051489, WO2014072481, WO2011103076, WO2012112188, WO2013075066, WO2015063611, WO2014179657 and WO2019075519.

In one example, the sCR1 variant is fused to albumin, a functional fragment or variant thereof at its C-terminus. For example, the sCR1 variant consists of an amino acid sequence corresponding to amino acids 42 to 1392 of SEQ ID NO: 1 or comprises an amino acid sequence corresponding to amino acids 42 to 1392 of SEQ ID NO: 1 (e.g., lacking amino acid residues 1393 to 1971 of SEQ ID NO: 1) and is fused to an albumin (e.g., serum albumin), a functional fragment or variant thereof at its C-terminus.

In one example, other proteins that are structurally or evolutionarily related to albumin may be used as half-life extending moieties, including, but not limited to alpha-fetoprotein (WO 2005024044; Beattie and Dugaiczyk, 20 Gene 415-422, 1982), afamin (Lichenstein et al. 269 (27) J. Biol. Chem. 18149-18154, 1994), and vitamin D binding protein (Cooke and David, 76 J. Clin. Invest. 2420-2424, 1985).

In one example, the half-life extending moiety is alpha-fetoprotein.

In one example, the half-life extending moiety is afamin.

In one example, the half-life extending moiety is vitamin D binding protein.

In one example, the half-life extending moiety is an immunoglobulin or functional fragment thereof. In one example, the immunoglobulin comprises an Fc region. For example, the Ig is an Fc domain or an Fc fragment and/or variant thereof. In one example, the Ig is a portion(s) of the immunoglobulin constant domain(s). In one example, the immunoglobulin is an antibody fragment that binds to albumin.

In one example, the half-life extending moiety is a polymer. Polymers suitable for use in the present disclosure will be apparent to the skilled person and include, for example, polyethylene glycol. In one example, the polymer comprises a mono- or poly- (e.g., 2-4) polyethylene glycol (PEG). In one example, the polymer is PEG.

In one example, the sCR1 variant of the present disclosure is conjugated to a further soluble complement inhibitor. For example, the further soluble complement inhibitor is selected from the group consisting of C1-inhibitor (C1-INH), Factor I, (fI), Factor H (fH), complement Factor H related protein (CFHR), C4b-binding protein (C4bp), soluble CD55 (decay accelerating factor (DAF)), soluble CD46 (membrane cofactor protein (MCP)), soluble CD59 (protectin), soluble complement receptor 2 (sCR2), TT30 (CR2-fH) and Cobra venom factor (CVF).

In one example, the further soluble complement inhibitor is a C1-inhibitor (C1-INH).

In one example, the further soluble complement inhibitor is Factor I (fI).

In one example, the further soluble complement inhibitor is Factor H (fH). In one example, the further soluble complement inhibitor is a complement Factor H related protein (CFHR). For example, the complement Factor H related protein is selected from the group consisting of CFHR1, CFHR2, CFHR3, CFHR4 and CFHR5.

In one example, the further soluble complement inhibitor is C4b-binding protein (C4bp).

In one example, the further soluble complement inhibitor is soluble CD55 (decay accelerating factor (DAF)).

In one example, the further soluble complement inhibitor is soluble CD46 (membrane cofactor protein (MCP)).

In one example, the further soluble complement inhibitor is soluble CD59 (protectin).

In one example, the further soluble complement inhibitor is soluble complement receptor 2 (sCR2).

In one example, the further soluble complement inhibitor is TT30 (CR2-fH).

In one example, the further soluble complement inhibitor is Cobra venom factor (CVF).

In one example, the sCR1 variant of the disclosure is a sCR1 variant glycoform. For example, the sCR1 variant glycoform is a sialylated sCR1 variant glycoform. In one example, the sCR1 variant glycoforms in the composition comprise sialylated glycans. For example, the sialylated sCR1 variant glycoform comprises at least one sialylated glycan (e.g., mono-, di-, tri- or tetra-sialylated glycans). In one example, the sialylated sCR1 variant glycoform is a sCR1 variant glycoform comprising one sialylated glycan (i.e., the sCR1 variant is mono-sialylated). In one example, the sialylated sCR1 variant glycoform comprises at least two sialylated glycans (e.g., di-, tri- or tetra-sialylated). In one example, the sialylated sCR1 variant glycoform is a sCR1 variant glycoform comprising two sialylated glycans (i.e., the sCR1 variant is di-sialylated). In one example, the sCR1 variant is a sCR1 variant glycoform comprising three sialylated glycans (i.e., the sCR1 variant is tri-sialylated). In one example, the sCR1 variant is a sCR1 variant glycoform comprising four sialylated glycans (i.e., the sCR1 variant is tetra-sialylated).

In one example, the sCR1 variant glycoform (i.e., comprising at least two sialylated glycans) has increased inhibitory activity in the classical pathway, the lectin pathway and/or alternative complement pathway compared to a sCR1 comprising a sequence set forth in SEQ ID NO: 2. The present disclosure provides a sCR1 variant glycoform (i.e., comprising at least two sialylated glycans) of the present disclosure conjugated to a half-life extending moiety or a further soluble complement inhibitor. In one example, the conjugated sCR1 variant glycoform of the present disclosure has a longer serum half-life compared to a sCR1 variant conjugate comprising a sCR1 set forth in SEQ ID NO: 2. Examples of increased serum half-life and assays for determining serum half-life are described herein and are to be taken to apply *mutatis mutandis* to this example of the disclosure.

The present disclosure also provides a composition comprising a sCR1 variant of the disclosure and a pharmaceutical carrier and/or excipient. In one example, the present disclosure also provides a composition comprising sCR1 variant glycoforms. For example, the composition comprises sialylated sCR1 variant glycoforms. In one example, at least 30% of the sCR1 variant glycoforms in the composition comprise sialylated glycans. For example, at least 30% of the sialylated sCR1 variant glycoforms comprise mono-, di-, tri- and/or tetra-sialylated glycans. For example, about 30%, or about 35%, or about 40%, or about 45%, or about 50%, or about 55%, or about 60%, or about 65%, or about 70%, or about 75% of the sCR1 variant glycoforms in the composition comprise sialylated glycans (e.g., mono-, di-, tri- and/or tetra-sialylated).

In one example, the composition comprises sialylated sCR1 variant glycoforms comprising at least two sialylated glycans (e.g., di-, tri- or tetra-sialylated glycans). In one example, at least about 30% of the sCR1 variant glycoforms in the composition comprise at least two sialylated glycans (e.g., di-, tri- or tetra-sialylated glycans). In one example, at least about 30% of the sCR1 variant glycoforms in the composition comprise di-, tri- and/or tetra-sialylated glycans. For example, about 30%, or about 35%, or about 40%, or about 45%, or about 50%, or about 55%, or about 60%, or about 65%, or about 70%, or about 75% of the sCR1 variant glycoforms in the composition comprise at least two sialylated glycans (e.g., di-, tri- or tetra-sialylated glycans). For example, about 30%, or about 35%, or about 40%, or about 45%, or about 50%, or about 55%, or about 60%, or about 65%, or about 70%, or about 75% of the sCR1 variant glycoforms in the composition comprise di-, tri- and/or tetra-sialylated glycans. The sCR1 variant consists of an amino acid sequence corresponding to amino acids 42 to 1392 of SEQ ID NO: 1 or comprises an amino acid sequence corresponding to amino acids 42 to 1392 of SEQ ID NO: 1 (e.g., lacking amino acid residues 1393 to 1971 of SEQ ID NO: 1).

In one example, the composition comprises sCR1 variant glycoforms comprising mono-sialylated glycans. In one example, about 20% of the sCR1 variant glycoforms in the composition comprise mono-sialylated glycans. For example, about 20%, or about 25%, or about 30%, or about 35%, or about 40% or about 45% of the sCR1 variant glycoforms in the composition comprise mono-sialylated glycans. In one example, about 22.5% to 25% of the sCR1 variant glycoforms in the composition comprise mono-sialylated glycans. In one example, about 35% to about 40% of the sCR1 variant glycoforms in the composition comprise mono-sialylated glycans. In one example, about 40% to about 45% of the sCR1 variant glycoforms in the composition comprise mono-sialylated glycans.

In one example, the composition comprises sCR1 variant glycoforms comprising di-sialylated glycans. In one example, at least about 15% of the sCR1 variant glycoforms in the composition comprise di-sialylated glycans. For example, about 15%, or about 17.5%, or about 20%, or about 22.5%, or about 25% of the sCR1 variant glycoforms in the composition comprise di-sialylated glycans. In one example, at least about 25% of the sCR1 variant glycoforms in the composition comprise di-sialylated glycans. For example, about 25%, or about 27.5%, or about 30%, or about 35%, or about 40%, or about 45%, or about 50%, or about 55%, or about 60% of the sCR1 variant glycoforms in the composition comprise di-sialylated glycans. In one example, about 17.5% to about 20% of the sCR1 variant glycoforms in the composition comprise di-sialylated glycans. In one example, about 25% to about 30% of the sCR1 variant glycoforms in the composition comprise di-sialylated glycans. In one example, about 40% to about 45% of the sCR1 variant glycoforms in the composition comprise di-sialylated glycans. The sCR1 variant consists of an amino acid sequence corresponding to amino acids 42 to 1392 of SEQ ID NO: 1 or comprises an amino acid sequence corresponding to amino acids 42 to 1392 of SEQ ID NO: 1 (e.g., lacking amino acid residues 1393 to 1971 of SEQ ID NO: 1).

In one example, the composition comprises sCR1 variant glycoforms comprising tri-sialylated glycans. In one example, at least about 1% of the sCR1 variant glycoforms in the composition comprise tri-sialylated glycans. For example, about 1%, or about 2%, or about 3%, or about 4%, or about 5%, or about 6%, or about 7%, or about 8%, or about 9%, or about 10%, or about 11%, or about 12%, or about 13%, or about 14%, or about 15% of the sCR1 variant glycoforms in the composition comprise tri-sialylated glycans. In one example, about 3.5% to about 4% of the sCR1 variant glycoforms in the composition comprise tri-sialylated glycans. In one example, about 8% to about 8.5% of the sCR1 variant glycoforms in the composition comprise tri-sialylated glycans. In one example, about 9% to about 9.5% of the sCR1 variant glycoforms in the composition comprise tri-sialylated glycans. The sCR1 variant consists of an amino acid sequence corresponding to amino acids 42 to 1392 of SEQ ID NO: 1 or comprises an amino acid sequence corresponding to amino acids 42 to 1392 of SEQ ID NO: 1 (e.g., lacking amino acid residues 1393 to 1971 of SEQ ID NO: 1).

In one example, the composition comprises sCR1 variant glycoforms comprising tetra-sialylated glycans. In one example, at least about 0.5% of the sCR1 variant glycoforms in the composition comprise tetra-sialylated glycans. For example, about 0.5%, or about 0.75%, or about 1%, or about 1.25%, or about 1.5%, or about 1.75%, or about 2%, or about 2.25%, or about 2.5%, or about 2.75%, or about 3%, or about 4%, or about 5%, or about 6%, or about 7%, or about 8%, or about 9%, or about 10% of the sCR1 variant glycoforms in the composition comprise tetra-sialylated glycans. In one example, about 0.5% of the sCR1 variant glycoforms in the composition comprise tetra-sialylated glycans. In one example, about 1.5% to about 2% of the sCR1 variant glycoforms in the composition comprise tetra-sialylated glycans. In one example, about 2% to about 2.5% of the sCR1 variant glycoforms in the composition comprise tetra-sialylated glycans. The sCR1 variant consists of an amino acid sequence corresponding to amino acids 42 to 1392 of SEQ ID NO: 1 or comprises an amino acid sequence corresponding to amino acids 42 to 1392 of SEQ ID NO: 1 (e.g., lacking amino acid residues 1393 to 1971 of SEQ ID NO: 1).

Methods of analysing the complex carbohydrate structure of the sCR1 variant of the present disclosure will be apparent to the skilled person and/or described herein.

Methods for producing the sCR1 variant glycoforms compositions of the disclosure will be apparent to the skilled person and include, for example expressing the sCR1 variant in a cell that recombinantly expresses and/or that overexpresses a sialyltransferase. For example, the sCR1 variant is expressed in a cell that recombinantly expresses and/or that overexpresses human ST3GAL3 (ST3 beta-galactoside alpha-2,3-sialyltransferase 3) and/or human B4GALT1 (human 01,4-galactosyltransferase). In another example, the sCR1 variant can be modified to include an additional glycosylation site to increase the level of glycosylation.

In one example, the sCR1 variant of the disclosure are expressed in a mammalian cell line. For example, the sCR1 variant is expressed in a mammalian cell line selected from the group consisting of Chinese hamster ovary (CHO) cells, human embryonic kidney cells (e.g., HEK293), baby hamster kidney (e.g., BHK-21) cells, murine myeloma cells (e.g., NS0, Sp2) and an amniocyte-derived cell such as CAP^{®} cells (e.g., CAP-Go. 1, CAP-Go.2).

In one example, the sCR1 variant is expressed in Chinese hamster ovary (CHO) cells.

In one example, the sCR1 variant is expressed in human embryonic kidney cells (e.g., HEK293).

In a further example, the sCR1 variant is expressed in amniocyte-derived cells, such as human amniocyte-derived cells, for example, CAP^{®} cells, for example, CAP^{®}-Go.1 or CAP^{®}-Go.2 cells. CAP^{®} cells suitable for use in any method of the present disclosure are described, for example, in Wissing *et al.,* (2015; BMC Proc., 9(Suppl 9):P12).

The present disclosure also provides a composition comprising a sCR1 conjugate of the disclosure and a pharmaceutical carrier and/or excipient.

In one example, the composition has increased serum half-life compared to a composition comprising a sCR1 conjugate comprising a sCR1 comprising a sequence set forth in SEQ ID NO: 2.

The present disclosure provides a soluble complement receptor type 1 (sCR1) variant for use in a method of inhibiting complement activity in a subject, the method comprising administering the sCR1 variant conjugate of the present disclosure, or the composition comprising the sCR1 variant.

The present disclosure also provides a soluble complement receptor type 1 (sCR1) variant for use in a method of treating or preventing a disease or condition in a subject, the method comprising administering the sCR1 variant conjugate of the present disclosure, or the composition comprising the sCR1 variant.

In one example, the present disclosure provides a sCR1 variant, or a sCR1 variant conjugate, or a composition comprising the sCR1 variant, for use in inhibiting complement activity in a subject.

In one example, the present disclosure provides a sCR1 variant, or a sCR1 variant conjugate, or a composition comprising the sCR1 variant, for use in treating or preventing a disease or condition in a subject.

In one example, the present disclosure provides a use of the sCR1 variant, or sCR1 variant conjugate, or the composition comprising the sCR1 variant of the present disclosure, in the manufacture of a medicament for inhibiting complement activity in a subject.

In one example, the present disclosure provides a use of the sCR1 variant, or sCR1 variant conjugate, or the composition comprising the sCR1 variant of the present disclosure, in the manufacture of a medicament for the treatment or prevention of a disease or condition in a subject.

In one example, the subject is in need of treatment with a sCR1 variant of the present disclosure (i.e., in need thereof).

In one example, the disease or condition is a complement mediated disorder. For example, the subject is suffering from, or at risk of a complement mediated disorder.

In one example, the subject suffers from a complement mediated disorder. In one example, the subject has been diagnosed as suffering from a complement mediated disorder. In one example, the subject is receiving treatment for a complement mediated disorder.

In one example of any method described herein, the sCR1 variant conjugate or composition comprising the sCR1 variant of the present disclosure is administered before or after the development of a complement mediated disorder. In one example of any method described herein, the sCR1 variant conjugate or composition comprising the sCR1 variant of the present disclosure is administered before the development of the complement mediated disorder. In one example of any method described herein, the sCR1 variant conjugate or composition comprising the sCR1 variant of the present disclosure is administered after the development of the complement mediated disorder.

In one example, the subject is at risk of developing a complement mediated disorder.

In one example, the sCR1 variant conjugate or composition comprising the sCR1 variant is administered before or after the onset of symptoms of a complement mediated disorder. In one example, the sCR1 variant conjugate or composition comprising the sCR1 variant is administered before the onset of symptoms of a complement mediated disorder. In one example, the sCR1 variant conjugate or composition comprising the sCR1 variant is administered after the onset of symptoms of a complement mediated disorder. In one example, the sCR1 variant conjugate or composition comprising the sCR1 variant of the present disclosure is administered at a dose that alleviates or reduces one or more of the symptoms of a complement mediated disorder.

Symptoms of a complement mediated disorder will be apparent to the skilled person and will be dependent on the condition. Exemplary symptoms of a complement mediated disorder include, for example:
- Recurring infection;
- Joint inflammation;
- Muscle weakness;
- Rash or discolouration of the skin;
- Edema, especially in the extremities (e.g., feet, hands, legs or arms) or eyes;
- Abdominal pain;
- Breathing difficulties;
- Nausea;
- Fatigue;
- Hematuria;
- Partial or complete paralysis; and
- Poor cognitive ability.

In one example, the complement mediated disorder is caused by primary dysregulation of the complement system, an autoimmune disorder, an acute injury and/or an inflammatory condition. For example, the complement mediated disorder is selected from the group consisting of hereditary angioedema, paroxysmal nocturnal haemoglobinuria (PNH), atypical haemolytic uraemic syndrome (aHUS), thrombocytopenic purpura (TTP), thrombotic microangiopathy, C3 glomerulopathy, membranoproliferative glomerulonephritis (including anti-Thy 1 glomerulonephritis, anti-conA diffuse proliferative glomerulonephritis and/or passive heymann nephritis), transplant rejection (including lung transplant (including Graft salvage or antibody mediated rejection) and/or solid organ transplantation (e.g., renal transplant (including antibody mediated rejection), neuromyelitis optica, multiple sclerosis, Guillain-Barré syndrome, myasthenia gravis (including autoimmune gyasthenia gravis, demyelinating allergic encephalomyelitis, IgG immune complex alveolitis, reverse passive arthus reaction), lupus nephritis (including acute lupus nephritis or chronic lupus nephritis), systemic lupus erythematosus (SLE), IgA nephropathy, rheumatoid arthritis, Crohn's disease, ulcerative colitis, autoimmune haemolytic anemia, pemphigus (including pemphigus vulgaris), pemphigoid (including bullous pemphigoid), anti-phospholipid syndrome, polytrauma, neurotrauma, haemodialysis, post-infection HUS, macular degeneration, uveitis, ANCA-associated vasculitis, atherosclerosis, mood disorders, asthma, chronic obstructive pulmonary disease (COPD), chronic inflammatory demyelinating polyneuropathy (CIDP), anaphylaxis, sepsis, cerebral malaria, psoriatic arthropathy, dermatomyositis, osteoarthritis, dementia, glaucoma, diabetic angiopathy, myocardial infarction, ischemic stroke (with or without reperfusion), haemorrhagic stroke, post-bypass surgery, anti-glomerular basement membrane (GBM) nephritis (or Goodpasture's syndrome), autoimmune epilepsy, dermatitis herpetiformis, eosinophilic granulomatosis with polyangiitis (EGPA; or Churg-Strauss syndrome), traumatic brain injury, somatic trauma, hidradenitis suppurativa, Sjögren's syndrome, Sjögren's syndrome vasculitis, trauma (including glycogen induced peritonitis, thermal trauma, nerve crush and/or closed head injury), ischemia reperfusion injury (IRI; including myocardial IRI, intestinal IRI, liver IRI and/or pancreatic IRI) and acute respiratory distress syndrome (or acute lung injury).

In one example, the complement mediated disorder is selected from the group consisting of transplant rejection (e.g., antibody mediated rejection), ischemia reperfusion injury before, during or after transplantation (including lung transplant and/or renal transplant), delayed graft function (including lung transplant and/or renal transplant), solid organ transplantation, neuromyelitis optica, myasthenia gravis, a glomerular pathology, lupus nephritis, IgA nephropathy, bullous pemphigoid, anti-phospholipid syndrome, uveitis, a neurological disorder, Parkinson's disease, Huntington's disease, cerebral infarction, motor neuron disease, autoimmune haemolytic anemia, ANCA-associated vasculitis, chronic inflammatory demyelinating polyneuropathy (CIDP) and anti-glomerular basement membrane (GBM) nephritis. In one example, the subject has a condition requiring prophylactic treatment.

In one example, the complement mediated disorder is selected from the group consisting of transplant rejection (including delayed graft function, graft salvage and antibody mediated rejection), solid organ transplantation, a nephropathy, ischemia-reperfusion injury, neuromyelitis optica, myasthenia gravis, a glomerular pathology, lupus nephritis (acute and chronic), IgA nephropathy, bullous pemphigoid, anti-phospholipid syndrome, uveitis, a neurological disorder, Parkinson's disease, Huntington's disease, cerebral infarction, motor neuron disease, autoimmune haemolytic anemia, ANCA-associated vasculitis chronic inflammatory demyelinating polyneuropathy, ischemic stroke (with and without reperfusion), traumatic brain injury, somatic trauma and anti-glomerular basement membrane (GBM) nephritis

In one example, the complement mediated disorder is transplant rejection (e.g., antibody mediated rejection).

In one example, the complement mediated disorder is solid organ transplantation.

In one example, the complement mediated disorder is ischemia reperfusion injury before, during or after transplantation (including lung transplant and/or renal transplant).

In one example, the complement mediated disorder is delayed graft function (including lung transplant and/or renal transplant).

In one example, the complement mediated disorder is neuromyelitis optica.

In one example, the complement mediated disorder is myasthenia gravis. For example, the myasthenia gravis is autoimmune gyasthenia gravis, demyelinating allergic encephalomyelitis, IgG immune complex alveolitis or reverse passive arthus reaction.

In one example, the complement mediated disorder is a glomerular pathology.

In one example, the complement mediated disorder is lupus nephritis. For example, the lupus nephritis is acute lupus nephritis or chronic lupus nephritis.

In one example, the complement mediated disorder is systemic lupus erythematosus (SLE). In one example, the complement mediated disorder is IgA nephropathy.

In one example, the complement mediated disorder is pemphigoid. For example, the pemphigoid is bullous pemphigoid.

In one example, the complement mediated disorder is anti-phospholipid syndrome.

In one example, the complement mediated disorder is uveitis.

In one example, the complement mediated disorder is a neurological disorder.

In one example, the complement mediated disorder is Parkinson's disease.

In one example, the complement mediated disorder is Huntington's disease.

In one example, the complement mediated disorder is cerebral infarction.

In one example, the complement mediated disorder is motor neuron disease.

In one example, the complement mediated disorder is autoimmune haemolytic anemia.

In one example, the complement mediated disorder is ANCA-associated vasculitis.

In one example, the complement mediated disorder is chronic inflammatory demyelinating polyneuropathy.

In one example, the complement mediated disorder is hereditary angioedema.

In one example, the complement mediated disorder is paroxysmal nocturnal haemoglobinuria (PNH).

In one example, the complement mediated disorder is atypical haemolytic uraemic syndrome (aHUS).

In one example, the complement mediated disorder is thrombocytopenic purpura (TTP).In one example, the complement mediated disorder is thrombotic microangiopathy.

In one example, the complement mediated disorder is C3 glomerulopathy.

In one example, the complement mediated disorder is membranoproliferative glomerulonephritis. For example, the glomerulonephritis is anti-Thy 1 glomerulonephritis, anti-conA diffuse proliferative glomerulonephritis and/or passive heymann nephritis.

In one example, the complement mediated disorder is transplant rejection. For example, the transplant lung transplant (including Graft salvage or antibody mediated rejection) and/or renal transplant (including antibody mediated rejection).

In one example, the complement mediated disorder is multiple sclerosis.

In one example, the complement mediated disorder is Guillain-Barré syndrome.

In one example, the complement mediated disorder is rheumatoid arthritis.

In one example, the complement mediated disorder is an inflammatory bowel disease. For example, the inflammatory bowel disease is Crohn's disease or ulcerative colitis.

In one example, the complement mediate disorder is pemphigus. For example, the pemphigus is pemphigus vulgaris.

In one example, the complement mediated disorder is polytrauma.

In one example, the complement mediated disorder is neurotrauma.

In one example, the complement mediated disorder is haemodialysis.

In one example, the complement mediated disorder is post-infection HUS.

In one example, the complement mediated disorder is macular degeneration.

In one example, the complement mediated disorder is atherosclerosis.

In one example, the complement mediated disorder is a mood disorder.

In one example, the complement mediated disorder is asthma.

In one example, the complement mediated disorder is chronic obstructive pulmonary disease (COPD).

In one example, the complement mediated disorder is chronic inflammatory demyelinating polyneuropathy (CIDP).

In one example, the complement mediated disorder is anaphylaxis.

In one example, the complement mediated disorder is sepsis.

In one example, the complement mediated disorder is cerebral malaria.

In one example, the complement mediated disorder is psoriatic arthropathy.

In one example, the complement mediated disorder is dermatomyositis.

In one example, the complement mediated disorder is osteoarthritis.

In one example, the complement mediated disorder is dementia.

In one example, the complement mediated disorder is glaucoma.

In one example, the complement mediated disorder is diabetic angiopathy.

In one example, the complement mediated disorder is myocardial infarction.

In one example, the complement mediated disorder is stroke. For example, the stroke is ischemic stroke (with or without reperfusion). In another example, the stroke is haemorrhagic stroke.

In one example, the complement mediated disorder is post-bypass surgery.

In one example, the complement mediated disorder is anti-glomerular basement membrane (GBM) nephritis (or Goodpasture's syndrome).

In one example, the complement mediated disorder is autoimmune epilepsy.

In one example, the complement mediated disorder is dermatitis herpetiformis.

In one example, the complement mediated disorder is eosinophilic granulomatosis with polyangiitis (EGPA; or Churg-Strauss syndrome).

In one example, the complement mediated disorder is traumatic brain injury.

In one example, the complement mediated disorder is trauma. For example, the trauma is somatic trauma. In one example, the trauma is glycogen induced peritonitis. In another example, the trauma is thermal trauma. In a further example, the trauma is nerve crush and/or closed head injury.

In one example, the complement mediated disorder is hidradenitis suppurativa.

In one example, the complement mediated disorder is Sjögren's syndrome. For example, the Sjögren's syndrome is Sjögren's syndrome vasculitis.

In one example, the complement mediated disorder is ischemia reperfusion injury (IRI). For example, the IRI is myocardial IRI, intestinal IRI, liver IRI and/or pancreatic IRI.

In one example, the complement mediated disorder is acute respiratory distress syndrome (or acute lung injury).

In one example, the sCR1 variant conjugate or composition comprising the sCR1 variant of the present disclosure is administered to the subject in an amount to reduce the severity of the complement mediated disorder in the subject.

In one example of any method described herein, the subject is a mammal, for example a primate such as a human.

Methods of treatment described herein can additionally comprise administering a further compound to reduce, treat or prevent the effect of the complement mediated disorder.

The present disclosure provides a kit comprising at least one sCR1 conjugate or composition comprising a sCR1 variant of the disclosure packaged with instructions for use in inhibiting complement activity in a subject. Optionally, the kit additionally comprises a further therapeutically active compound or drug.

The present disclosure further provides a kit comprising at least one sCR1 conjugate or composition comprising a sCR1 variant of the disclosure packaged with instructions for use in treating or preventing a complement mediated disorder in a subject. Optionally, the kit additionally comprises a further therapeutically active compound or drug.

The present disclosure also provides a kit comprising at least one sCR1 variant conjugate or composition comprising a sCR1 variant of the disclosure packaged with instructions to administer the conjugate or composition to a subject who is suffering from or at risk of suffering from a complement mediated disorder, optionally, in combination with a further therapeutically active compound or drug.

Exemplary effects of sCR1 variant conjugates or compositions of the present disclosure are described herein and are to be taken to apply *mutatis mutandis* to the examples of the disclosure set out in the previous four paragraphs.

### BRIEF DESCRIPTION OF FIGURES

**Figure 1** is a graphical representation showing the effect of sialylation of sCR1(1392)-8His on plasma half-life.
**Figure 2** is a graphical representation showing the effect of sCR1(1392)-8His treatment in an *in vivo* model of anti-GBM glomerulonephritis.
**Figure 3** is a graphical representation showing the effect of sCR1(1392)-8His^{SIA} treatment in an *in vivo* model of anti-GBM glomerulonephritis.

### KEY TO SEQUENCE LISTING

| | |
|---|---|
| SEQ ID NO: 1 | amino acid sequence of soluble complement receptor 1 (sCR1) with the N-terminal endogenous human CR1 signal peptide |
| SEQ ID NO: 2 | amino acid sequence of mature soluble complement receptor 1 (sCR1(1971)) lacking the N-terminal endogenous human CR1 signal peptide |
| SEQ ID NO: 3 | amino acid sequence of truncated mature soluble complement receptor 1 (sCR1(1392)) lacking the N-terminal endogenous human CR1 signal peptide |
| SEQ ID NO: 4 | amino acid sequence of truncated mature soluble complement receptor 1 (sCR1(939)) lacking the N-terminal endogenous human CR1 signal peptide |
| SEQ ID NO: 5 | amino acid sequence of truncated mature soluble complement receptor 1 (sCR1(490-1392)) |
| SEQ ID NO: 6 | amino acid sequence of truncated mature soluble complement receptor 1 (sCR1(490-1971)) |
| SEQ ID NO: 7 | amino acid sequence of truncated mature soluble complement receptor 1 (sCR1(234)) lacking the N-terminal endogenous human CR1 signal peptide |
| SEQ ID NO: 8 | amino acid sequence of truncated mature soluble complement receptor 1 (sCR1(489)) lacking the N-terminal endogenous human CR1 signal peptide |
| SEQ ID NO: 9 | amino acid sequence of truncated mature soluble complement receptor 1 (sCR1(940-1971)) |
| SEQ ID NO: 10 | amino acid sequence of truncated mature soluble complement receptor 1 (sCR1(490-939)) |
| SEQ ID NO: 11 | amino acid sequence of truncated mature soluble complement receptor 1 (sCR1(940-1392)) |
| SEQ ID NO: 12 | amino acid sequence of truncated mature soluble complement receptor 1 (sCR1(1393-1971)) |
| SEQ ID NO: 13 | amino acid sequence of sCR1 LHR-A |
| SEQ ID NO: 14 | amino acid sequence of sCR1 LHR-B |
| SEQ ID NO: 15 | amino acid sequence of sCR1 LHR-C |
| SEQ ID NO: 16 | amino acid sequence of sCR1 LHR-D |
| SEQ ID NO: 17 | 8xHis tag |
| SEQ ID NO: 18 | amino acid sequence of endogenous signal peptide |
| SEQ ID NO: 19 | amino acid sequence of exogenous signal peptide |
| SEQ ID NO: 20 | amino acid sequence of His tagged soluble complement receptor 1 (sCR1(1971)-8His) with N-terminal endogenous signal peptide |
| SEQ ID NO: 21 | amino acid sequence of His tagged truncated soluble complement receptor 1 (sCR1(1392)-8His) with N-terminal endogenous signal peptide |
| SEQ ID NO: 22 | amino acid sequence of truncated mature soluble complement receptor 1 (sCR1(939)-8His) with N-terminal endogenous signal peptide |
| SEQ ID NO: 23 | amino acid sequence of His tagged truncated soluble complement receptor 1 (sCR1(490-1392)-8His) with N-terminal exogenous signal peptide |
| SEQ ID NO: 24 | amino acid sequence of His tagged truncated soluble complement receptor 1 (sCR1(490-1971)-8His) with N-terminal exogenous signal peptide |
| SEQ ID NO: 25 | amino acid sequence of His tagged truncated soluble complement receptor 1 (sCR1(234)-8His) with N-terminal endogenous signal peptide |
| SEQ ID NO: 26 | amino acid sequence of His tagged truncated soluble complement receptor 1 (sCR1(489)-8His) with N-terminal endogenous signal peptide |
| SEQ ID NO: 27 | amino acid sequence of His tagged truncated soluble complement receptor 1 (sCR1(940-1971)-8His) with N-terminal exogenous signal peptide |
| SEQ ID NO: 28 | amino acid sequence of His tagged truncated soluble complement receptor 1 (sCR1(490-939)-8His) with N-terminal exogenous signal peptide |
| SEQ ID NO: 29 | amino acid sequence of His tagged truncated soluble complement receptor 1 (sCR1(940-1392)-8His) with N-terminal exogenous signal peptide |
| SEQ ID NO: 30 | amino acid sequence of His tagged truncated soluble complement receptor 1 (sCR1(1393-1971)-8His) with N-terminal exogenous signal peptide |
| SEQ ID NO: 31 | GS13 Linker |
| SEQ ID NO: 32 | amino acid sequence of mature human serum albumin |
| SEQ ID NO: 33 | amino acid sequence of IgG₁ Fc |
| SEQ ID NO: 34 | amino acid sequence of IgG₄ Fc |
| SEQ ID NO: 35 | GS30 Linker |
| SEQ ID NO: 36 | amino acid sequence of exogenous HSA signal peptide |
| SEQ ID NO: 37 | amino acid sequence of exogenous signal peptide |
| SEQ ID NO: 38 | amino acid sequence of soluble complement receptor 1 conjugated to HSA (sCR1(1971)-GS13-HSA) with N-terminal endogenous signal peptide |
| SEQ ID NO: 39 | amino acid sequence of soluble complement receptor 1 conjugated to HSA (HSA-GS13-sCR1(1971)) with N-terminal HSA signal peptide and pro-peptide |
| SEQ ID NO: 40 | amino acid sequence of soluble complement receptor 1 conjugated to IgG₄ Fc (sCR1(1971)-IgG₄ Fc) with N-terminal endogenous signal peptide |
| SEQ ID NO: 41 | amino acid sequence of soluble complement receptor 1 conjugated to IgG₄ Fc (IgG₄ Fc-sCR1(1971)) with N-terminal exogenous signal peptide |
| SEQ ID NO: 42 | amino acid sequence of HSA pro-peptide |
| SEQ ID NO: 43 | amino acid sequence of truncated mature soluble complement receptor 1 conjugated to HSA (sCR1(1392)-GS13-HSA) with N-terminal endogenous signal peptide |
| SEQ ID NO: 44 | amino acid sequence of truncated mature soluble complement receptor 1 conjugated to HSA (HSA-GS13-sCR1(1392)) with N-terminal exogenous signal peptide |
| SEQ ID NO: 45 | amino acid sequence of truncated mature soluble complement receptor 1 conjugated to IgG₁ Fc (sCR1(1392)-IgG₁ Fc) with N-terminal endogenous signal peptide |
| SEQ ID NO: 46 | amino acid sequence of truncated mature soluble complement receptor 1 conjugated to IgG₄ Fc (sCR1(1392)-IgG₄ Fc) with N-terminal endogenous signal peptide |
| SEQ ID NO: 47 | amino acid sequence of truncated mature soluble complement receptor 1 conjugated to IgG₄ Fc (IgG₄ Fc-sCR1(1392)) with N-terminal exogenous signal peptide |
| SEQ ID NO: 48 | amino acid sequence of truncated mature soluble complement receptor 1 conjugated to HSA (sCR1(939)-GS13-HSA) with N-terminal endogenous signal peptide |
| SEQ ID NO: 49 | amino acid sequence of truncated mature soluble complement receptor 1 conjugated to IgG₄ Fc (sCR1(939)-IgG₄ Fc)) with N-terminal endogenous signal peptide |
| SEQ ID NO: 50 | amino acid sequence of truncated mature soluble complement receptor 1 conjugated to IgG₄ Fc (IgG₄ Fc-sCR1(939)) with N-terminal exogenous signal peptide |
| SEQ ID NO: 51 | amino acid sequence of truncated mature soluble complement receptor 1 conjugated to HSA (sCR1(1392)-HSA) |

### DETAILED DESCRIPTION

### General

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or groups of compositions of matter.

Those skilled in the art will appreciate that the present disclosure is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

The present disclosure is not to be limited in scope by the specific examples described herein, which are intended for the purpose of exemplification only. Functionally-equivalent products, compositions and methods are clearly within the scope of the present disclosure. Any references to methods of inhibiting complement activity in a subject in this description are to be interpreted as references to soluble complement receptor type 1 (sCR1) variants of the present invention for use in those methods.

Any example of the present disclosure herein shall be taken to apply *mutatis mutandis* to any other example of the disclosure unless specifically stated otherwise. Stated another way, any specific example of the present disclosure may be combined with any other specific example of the disclosure (except where mutually exclusive).

Any example of the present disclosure disclosing a specific feature or group of features or method or method steps will be taken to provide explicit support for disclaiming the specific feature or group of features or method or method steps.

Unless specifically defined otherwise, all technical and scientific terms used herein shall be taken to have the same meaning as commonly understood by one of ordinary skill in the art (for example, in cell culture, molecular genetics, immunology, immunohistochemistry, protein chemistry, and biochemistry).

Unless otherwise indicated, the recombinant protein, cell culture, and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al. Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

The description and definitions of variable regions and parts thereof, immunoglobulins, antibodies and fragments thereof herein may be further clarified by the discussion in Kabat Sequences of Proteins of Immunological Interest, National Institutes of Health, Bethesda, Md., 1987 and 1991, Bork et al., J Mol. Biol. 242, 309-320, 1994, Chothia and Lesk J. Mol Biol. 196:901 -917, 1987, Chothia et al. Nature 342, 877-883, 1989 and/or or Al-Lazikani et al., J Mol Biol 273, 927-948, 1997.

The term "and/or", e.g., "X and/or Y" shall be understood to mean either "X and Y" or "X or Y" and shall be taken to provide explicit support for both meanings or for either meaning. Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

As used herein the term "derived from" shall be taken to indicate that a specified integer may be obtained from a particular source albeit not necessarily directly from that source.

### Selected Definitions

Complement receptor type 1 (CR1), also known as C3b/C4b receptor or CD35 is a member of the family of regulators of complement activation. CR1 is present on the membranes of erythrocytes, monocytes/macrophages, granulocytes, B cells, some T cells, splenic follicular dendritic cells, and glomerular podocytes, and mediates cellular binding to particles and immune complexes that have activated complement. The encoded protein has a 41 amino acid signal peptide, an extracellular domain of 1930 residues, a 25 residue transmembrane domain and a 43 amino acid C-terminal cytoplasmic region. For the purposes of nomenclature only and not limitation an exemplary sequence of human CR1 is set out in GenBank Accession no. NP_000564.

Soluble complement receptor type 1 (sCR1) is naturally produced by cleavage of cell surface CR1 and plays a role in the control of complement activation at sites of inflammation. It should be understood that reference to "sCR1" refers to truncated CR1, which lacks the transmembrane and cytoplasmic domains. For the purposes of nomenclature only and not limitation an exemplary sequence of human sCR1 is set out in SEQ ID NO: 1. Positions of amino acids are referred to herein by reference to sCR1 protein consisting of 1971 amino acids (e.g., as set out in SEQ ID NO: 1). Full length sCR1 comprises four long homologous repeat (LHR) regions, i.e., LHR-A, B, C and D. LHR regions may be defined with reference to human sCR1 (as set forth in SEQ ID NO: 1). For example, LHR-A comprises amino acids 42 to 489 of SEQ ID NO: 1, LHR-B comprises amino acids 490 to 939 of SEQ ID NO: 1, LHR-C comprises amino acids 940 to 1392 of SEQ ID NO: 1 and LHR-D comprises amino acids 1393 to 1971 of SEQ ID NO: 1. Each LHR comprises short consensus repeat (SCR) sequences with a total of 30 SCR sequences, each having 60 to 70 amino acids. For example, LHR-A comprises SCRs 1 to 7 (corresponding to amino acids 42 to 489 of SEQ ID NO: 1), LHR-B comprises SCRs 8 to 14 (corresponding to amino acids 491 to 939 of SEQ ID NO: 1), LHR-C comprises SCRs 15 to 21 (corresponding to amino acids 941 to 1389 of SEQ ID NO: 1), and LHR-D comprises SCRs 22 to 28 (corresponding to amino acids 1394 to 1842 of SEQ ID NO: 1) and SCRs 29 to 30 (corresponding to amino acids 1846 to 1967 of SEQ ID NO: 1). A sequence of mature human sCR1 lacks the N-terminal signal peptide corresponding to amino acids 1 to 41 of SEQ ID NO: 1. For example, a sequence of mature human sCR1 (i.e., lacking the N-terminal signal peptide) is set forth in SEQ ID NO: 2.

The sequence of sCR1 from other species can be determined using sequences provided herein and/or in publicly available databases and/or determined using standard techniques (e.g., as described in Ausubel et al., (editors), Current Protocols in Molecular Biology, Greene Pub. Associates and Wiley-Interscience (1988, including all updates until present) or Sambrook et al., Molecular Cloning: A Laboratory Manual, Cold Spring Harbor Laboratory Press (1989)). As used herein the phrase "corresponding to" in reference to the position of an amino acid in SEQ ID NO: 1 should be understood as reference to an amino acid residue or position within a sCR1 sequence, and not necessarily a sequence comprising SEQ ID NO: 1. For example, reference to "a position corresponding to amino acids 42 to 939 of SEQ ID NO: 1" in a sCR1 sequence comprising a 41 amino acid N-terminal truncation (i.e., mature sCR1) would necessarily refer to amino acids at position 1 to 898. In one example, the sCR1 comprises a sequence set forth in SEQ ID NO: 1.

As used herein, the term "variant" refers to a sCR1 which has undergone deletion or truncation of one or more amino acids using well known techniques.

As used herein, the term "inhibit(s)" or "inhibiting" in the context of complement activity shall be understood to mean that the sCR1 variant of the present disclosure reduces or decrease the level of complement activity. It will be apparent from the foregoing that the sCR1 variant of the present disclosure need not completely inhibit complement activity, rather it need only reduce activity by a statistically significant amount, for example, by at least about 10%, or about 20%, or about 30%, or about 40%, or about 50%, or about 60%, or about 70%, or about 80%, or about 90%, or about 95%. Methods for determining inhibition of complement activity are known in the art and/or described herein.

As used herein, the term "complement inhibitor" shall be understood to refer to a compound that reduces or decreases the level of complement activity either directly or indirectly, including for example, by inhibition, blocking, degradation and consumption of complement compounds. In one example of any method described herein, the complement inhibitor decreases the level of complement activity directly. It will be apparent from the foregoing that the complement inhibitors of the present disclosure need not completely inhibit complement activity, rather need only reduce activity by a statistically significant amount, for example, by at least about 10%, or about 20%, or about 30%, or about 40%, or about 50%, or about 60%, or about 70%, or about 80%, or about 90%, or about 95%. Methods for determining inhibition of complement activity are known in the art and/or described herein.

The term "half-life extending moiety" as used herein, shall be understood to refer to a polypeptide fusion partner that may increase the half-life of the sCR1 variant of the present disclosure *in vivo* in a subject. Exemplary half-life extending moieties include albumin, antibody Fc regions and polymers.

As used herein, the term "serum half-life" or "plasma half-life" in the context of the present disclosure refers to the period of time required for the concentration or amount of sCR1 in the serum to be reduced by 50% (i.e., one half) for example due to degradation and/or clearance or sequestration by natural mechanisms. The skilled person would recognise that the serum half-life of sCR1 in a subject is dependent on various physiological conditions (e.g., health status, body size/weight). In a healthy human subject, the serum half-life of sCR1 is approximately 70 hours (3 days). Methods for determining the serum half-life of sCR1 are known in the art and include, for example, pharmacokinetic analysis. For the purposes of the present disclosure, an "increase" or "enhanced" serum half-life refers to an elevation or increase in time taken for the serum concentration of the sCR1 variant to be reduced by 50%, compared to a sCR1 set forth in SEQ ID NO: 2.

The term "recombinant" shall be understood to mean the product of artificial genetic recombination. A recombinant protein also encompasses a protein expressed by artificial recombinant means when it is within a cell, tissue or subject, e.g., in which it is expressed. The term "protein" shall be taken to include a single polypeptide chain, i.e., a series of contiguous amino acids linked by peptide bonds or a series of polypeptide chains covalently or non-covalently linked to one another (i.e., a polypeptide complex). For example, the series of polypeptide chains can be covalently linked using a suitable chemical or a disulfide bond. Examples of non-covalent bonds include hydrogen bonds, ionic bonds, Van der Waals forces, and hydrophobic interactions.

The term "polypeptide" or "polypeptide chain" will be understood from the foregoing paragraph to mean a series of contiguous amino acids linked by peptide bonds.

The phrase "conservative amino acid substitution" refers to replacement or substitution of an amino acid residue with an amino acid residue having a similar side chain and/or hydropathicity and/or hydrophilicity. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), *β*-branched side chains (e.g., threonine, valine, isoleucine) and aromatic side chains (e.g., tyrosine, phenylalanine, tryptophan, histidine). Hydropathic indices are described, for example in Kyte and Doolittle J. Mol. Biol., 157: 105-132, 1982 and hydrophylic indices are described in, e.g., US4554101.

As used herein, the term "condition" refers to a disruption of or interference with normal function, and is not to be limited to any specific condition, and will include diseases or disorders.

As used herein, a subject "at risk" of developing a disease or condition or relapse thereof or relapsing may or may not have detectable disease or symptoms of disease, and may or may not have displayed detectable disease or symptoms of disease prior to the treatment according to the present disclosure. "At risk" denotes that a subject has one or more risk factors, which are measurable parameters that correlate with development of the disease or condition, as known in the art and/or described herein.

As used herein, the terms "treating", "treat" or "treatment" include administering a serum albumin variant conjugate described herein to thereby reduce or eliminate at least one symptom of a specified disease or condition or to slow progression of the disease or condition.

As used herein, the term "preventing", "prevent" or "prevention" includes providing prophylaxis with respect to occurrence or recurrence of a specified disease or condition in an individual. An individual may be predisposed to or at risk of developing the disease or disease relapse but has not yet been diagnosed with the disease or the relapse.

As used herein, the term "subject" shall be taken to mean any animal including humans, for example a mammal. Exemplary subjects include but are not limited to humans and non-human primates. For example, the subject is a human.

### Inhibiting Complement Activity

The present disclosure provides, for example, a method of inhibiting complement activity in a subject comprising administering to the subject a soluble complement receptor type 1 (sCR1) variant of the present disclosure.

The present disclosure also provides a method of treating or preventing a disease or condition in a subject, the method comprising administering the sCR1 variant, or sCR1 variant conjugate or composition comprising the sCR1 variant of the present disclosure to a subject. In one example, the present disclosure provides a method of treating a disease or condition in a subject in need thereof.

The present disclosure also provides for use of a sCR1 variant, or sCR1 variant conjugate or composition comprising the sCR1 variant of the present disclosure for treating or preventing a disease or condition in a subject. In one example, the present disclosure provides for use of a serum albumin conjugate of the present disclosure for treating a disease or condition in a subject in need thereof.

In one example, the method comprises inhibiting activity in the classical pathway, the lectin pathway and/or the alternative complement pathway. For example, the method comprises administering a sCR1 variant of the present disclosure to inhibit activation of the classical complement pathway. In another example, the method comprises administering a sCR1 variant of the present disclosure to inhibit activation of the lectin pathway. In a further example, the method comprises administering a sCR1 variant of the present disclosure to inhibit activation of the alternative complement pathway.

In one example, the method comprises inhibiting activity in the extrinsic complement pathway. For example, the method comprises administering a sCR1 variant of the present disclosure to inhibit activation of the extrinsic complement pathway.

In one example, the disease or condition is a complement mediated disorder.

In one example, the subject suffers from a complement mediated disorder. The complement mediated disorder can be inherited or acquired.

In one example, the complement mediated disorder is selected from the group consisting of transplant rejection (including delayed graft function, graft salvage and antibody mediated rejection), a solid organ transplantation, a nephropathy, ischemia-reperfusion injury, neuromyelitis optica, myasthenia gravis, a glomerular pathology, lupus nephritis (acute and chronic), IgA nephropathy, bullous pemphigoid, anti-phospholipid syndrome, uveitis, a neurological disorder, Parkinson's disease, Huntington's disease, cerebral infarction, motor neuron disease, autoimmune haemolytic anemia, ANCA-associated vasculitis, chronic inflammatory demyelinating polyneuropathy, ischemic stroke (with and without reperfusion), traumatic brain injury, somatic trauma and anti-glomerular basement membrane (GBM) nephritis.

In one example, the complement-mediated disorder is a primary dysregulation, such as a hereditary angioedema, paroxysmal nocturnal hemoglobinuria, atypical hemolytic uremic syndrome (aHUS), thrombotic thrombocytopenic purpura (TTP), thrombotic microangiopathy, C3 glomerulopathy, membranoproliferative glomerulonephritis or transplant rejection (including delayed graft function, graft salvage and antibody mediated rejection).

In one example, the complement-mediated disorder is an autoimmune condition, such as neuromyelitis optica, multiple sclerosis, myasthenia gravis, Guillain-Barre syndrome, myasthenia gravis, lupus nephritis (acute and chronic), IgA nephropathy, rheumatoid arthritis, Crohn's disease, ulcerative colitis, autoimmune hemolytic anemia, pemphigus, pemphigoid (including bullous pemphigoid) chronic inflammatory demyelinating polyneuropathy (CIDP), anti-glomerular basement membrane (GBM) nephritis or anti-phospholipid syndrome.

In one example, the complement mediated disorder is an acute injury, such as polytrauma, neurotrauma, hemodialysis, traumatic brain injury, somatic trauma or post infection HUS.

In one example, the complement-mediated disorder is an inflammatory condition such as macular degeneration, uveitis, ANCA-associated vasculitis, atherosclerosis, asthma, COPD, sepsis, acute respiratory distress syndrome, cerebral malaria, psoriatic arthropathy or dermatomyositis.

In one example, the complement-mediated disorder is a degenerative condition such as osteoarthritis, dementia, glaucoma, a neurological disorder, Parkinson's disease, Huntington's disease, motor neuron disease or diabetic angiopathy.

In one example, the complement-mediated disorder is an ischemia-reperfusion condition/injury, e.g., as occurs in organ transplantation, or post-surgery or following stroke or myocardial infarction.

Methods for diagnosis of a complement mediated disorder will be readily apparent to the skilled person and include, for example, haemolytic classical complement pathway (CH-50) test, haemolytic alternative complement pathway (AP-50) test, screening for immune complex diseases, antinuclear serology to test for lupus, urinalysis and complete blood count (CBC).

In one example, the subject is at risk of developing a complement mediated disorder. A subject is at risk if he or she has a higher risk of developing a complement mediated disorder than a control population. The control population may include one or more subjects selected at random from the general population (e.g., matched by age, gender, race and/or ethnicity) who have not suffered from or have a family history of a complement mediated disorder. A subject can be considered at risk for a complement mediated disorder if a "risk factor" associated with a complement mediated disorder is found to be associated with that subject. A risk factor can include any activity, trait, event or property associated with a given disorder, for example, through statistical or epidemiological studies on a population of subjects. A subject can thus be classified as being at risk for a complement mediated disorder even if studies identifying the underlying risk factors did not include the subject specifically.

In one example, the subject is at risk of developing a complement mediated disorder and the sCR1 variant is administered before or after the onset of symptoms of a complement mediated disorder. In one example, the sCR1 variant is administered before the onset of symptoms of a complement mediated disorder. In one example, the sCR1 is administered after the onset of symptoms of a complement mediated disorder. In one example, the sCR1 variant of the present disclosure is administered at a dose that alleviates or reduces one or more of the symptoms of a complement mediated disorder in a subject at risk.

The methods of the present disclosure can be readily applied to any form of complement mediated disorder in a subject.

In one example, a method of the disclosure reduces any symptom of a complement mediated disorder known in the art and/or described herein.

As will be apparent to the skilled person a "reduction" in a symptom of a complement mediated disorder in a subject will be comparative to another subject who also suffers from a complement mediated disorder but who has not received treatment with a method described herein. This does not necessarily require a side-by-side comparison of two subjects. Rather population data can be relied upon. For example, a population of subjects suffering from a complement mediated disorder who have not received treatment with a method described herein (optionally, a population of similar subjects to the treated subject, e.g., age, weight, race) are assessed and the mean values are compared to results of a subject or population of subjects treated with a method described herein.

In the case of a complement-mediated condition that is an ischemia-reperfusion injury due to or associated with organ transplantation, the sCR1 variant of the disclosure or composition comprising the sCR1 variant can be administered before, during or after transplantation. In some examples, the sCR1 variant or composition is administered to an organ transplantation donor. In other examples, the sCR1 variant or composition is administered to the subject, wherein the subject is an organ transplantation recipient. In one example, the sCR1 variant or composition is administered to a harvested organ *ex vivo*, prior to organ transplantation. For example, the harvested organ can be perfused or infused with a solution comprising the sCR1 variant or composition prior to transplantation.

In one example, the organ transplantation is solid organ transplantation. For example, the solid organ transplantation is lung transplantation.

It will be apparent to the skilled person from the foregoing, that the present disclosure provides a method of organ transplantation or for improving outcome of an organ transplantation or improving function of a transplanted organ or for preventing delayed graft function, the method comprising administering a sCR1 variant or composition to an organ transplant donor prior to collection of the organ; collecting the organ and transplanting the organ into an organ transplant recipient.

The present disclosure also provides a method for preparing a transplant organ from an organ donor to improve organ function in an organ transplant recipient, the method comprising administering to the organ donor a sCR1 variant or composition prior to collection of the organ.

The present disclosure additionally provides a method for preventing organ transplant rejection, the method comprising administering to an organ donor a sCR1 variant or composition prior to collection of the organ, collecting the organ and transplanting the organ into an organ transplant recipient.

In some examples, the method additionally comprises administering the sCR1 variant or composition to the organ transplant recipient. For example, the sCR1 variant or composition is administered to the organ transplant recipient before the transplant or at the time of transplanting the organ (i.e., during transplantation).

The present disclosure also provides a method of organ transplantation or for improving outcome of an organ transplantation or improving function of a transplanted organ or for preventing delayed graft function, the method comprising administering a sCR1 variant or composition to an organ transplant recipient prior to transplanting the organ and then transplanting the organ into the organ transplant recipient.

In one example, the organ transplant donor is brain dead. For example, the organ donor is alive by virtue of life support but is brain dead.

In one example of the disclosure, the sCR1 variant or composition is administered before reperfusion, for example, in the case of an organ transplant, the sCR1 variant or composition is administered to an organ transplant recipient prior to reperfusion of the transplanted organ (e.g., the sCR1 variant or composition is administered prior to the transplantation or during the transplantation but before reperfusion).

In the case of administration to a brain dead donor, the sCR1 variant or composition can be administered at any time between brain death and organ collection. In some examples, the sCR1 variant or composition is administered to a harvested organ *ex vivo*, prior to organ transplantation. For example, the harvested organ can be perfused or infused with a solution comprising the sCR1 variant or composition prior to transplantation.

### Soluble Complement Receptor Type 1 Variants

The present disclosure provides a sCR1 variant for use in any method described herein.

In one example, the present disclosure provides a sCR1 variant that has improved or increased complement inhibitory activity compared to a sequence set forth in SEQ ID NO: 2. The inventors have determined that a sCR1 variant comprising residues 42 to 1392 of SEQ ID NO: 1 has improved and/or increased complement inhibitory activity.

The present disclosure provides a soluble complement receptor type 1 (sCR1) variant for use in a method of inhibiting complement activity in a subject, the method comprising administering a soluble complement receptor type 1 (sCR1) variant to the subject, the sCR1 variant comprising an amino acid sequence corresponding to amino acids 42 to 1392 of SEQ ID NO: 1, wherein the sCR1 variant comprises long homologous repeat (LHR) regions LHR-A, LHR-B and LHR-C, but is lacking LHR-D.

For example, the inventors have identified amino acid residues in a sequence set forth in SEQ ID NO: 1 that can be deleted without loss of function or that result in improved function. In one example, the sCR1 variant comprises deletion of between 489 and 1073 amino acids compared to a sequence set forth in SEQ ID NO: 1. For example, the sCR1 variant comprises deletion of 489 or 620 or 1068 or 1073 amino acids compared to a sequence set forth in SEQ ID NO: 1.

In one example, the present disclosure provides a truncated sCR1 comprising between 898 and 1482 amino acids compared to a sequence set forth in SEQ ID NO: 1. For example, the truncated sCR1 comprises 898 or 903 or 1351 or 1482 amino acids compared to a sequence set forth in SEQ ID NO: 1.

The sCR1 variant of the present disclosure comprises a variant of a sequence set forth in SEQ ID NO: 1, wherein the variant sequence comprises an amino acid sequence corresponding to amino acids 42 to 1392 of SEQ ID NO: 1.

In one example, the sCR1 variant of the present disclosure does not comprise or consist of a sequence set forth in SEQ ID NO: 1 and/or SEQ ID NO: 2.

In one example, the sCR1 variant of the present disclosure does not comprise an amino acid sequence corresponding to amino acids 1 to 41 of SEQ ID NO: 1.

In one example, the sCR1 variant of the present disclosure does not comprise an amino acid sequence corresponding to amino acids 940 to 1971 of SEQ ID NO: 1.

In one example, the sCR1 variant of the present disclosure does not comprise an amino acid sequence corresponding to amino acids 1393 to 1971 of SEQ ID NO: 1.

In one example, the sCR1 variant of the present disclosure does not comprise an amino acid sequence corresponding to amino acids 1 to 489 of SEQ ID NO: 1.

In one example, the sCR1 variant is monomeric (i.e., one copy of the sCR1 variant).

In one example, the sCR1 variant is dimeric, or dimerized (i.e., two copies of a sCR1 variant are linked in a fusion protein).

In one example, the sCR1 variant is multimeric, or multimerized (i.e., multiple copies of a sCR1 variant are linked in a fusion protein).

Methods for achieving dimerization or multimerization of the sCR1 variant are known in the art and/or described herein and include, for example, direct conjugation between the two or more sCR1 variants or indirect binding (e.g., by virtue of a linker between the two or more sCR1 variants). In one example, the dimerization or multimerization is formed by a chemical conjugation (e.g., by a disulphide bond or cystine knot) or by genetic fusion.

In one example, two or more of the same sCR1 variant are fused (i.e., expressed as a fusion protein).

In one example, two or more different sCR1 variants are fused (i.e., expressed as a fusion protein).

In one example, the dimerized or multimerized sCR1 variant comprises a linker between the sCR1 variants.

In one example, the disclosure provides a multimeric protein comprising two or more sCR1 variants comprising a multimerization domain, wherein the multimerization domains interact to form the multimeric protein.

In one example, each sCR1 variant in the multimeric protein comprises one sCR1 variant. In another example, one or more sCR1 variants in the multimeric protein comprises two or more sCR1 variants, e.g., the variants are linked in a fusion protein.

In one example, the multimerization domain comprises an immunoglobulin hinge domain.

In one example, the multimerization domain is a leucine zipper domain, a cystine knot or an antibody Fc region. For example, the multimerization domain is a leucine zipper domain. Suitable leucine zipper polypeptides will be known in the art and include c-Jun and c-Fos leucine zipper domains. Leucine zipper fusions are described in Riley et al., Protein Eng. (1996), which is incorporated herein by reference. In another example, the multimerization domain is a cystine knot. For example, the cystine knot comprises up to 60 amino acids in length including a core domain of three or more interwoven disulfide bonds. In a further example, the multimerization domain is an antibody Fc region (e.g., as described herein).

In one example, the multimerized sCR1 variant is linear.

In one example, the multimerized sCR1 variant is circular. For example, the multimerized sCR1 variant can comprise a sortase enzyme cleavage site, as described in Popp, M.W. et al. PNAS (2011), incorporated herein by reference.

In one example, the sCR1 variant for use in the present disclosure comprises at least two sialylated glycans (e.g., di-, tri- or tetra-sialylated glycans). For example, a composition for use in any method described herein comprises a sialylated sCR1 variant glycoform. In one example, a sialylated sCR1 variant glycoform for use in any method described herein comprises di-, tri- or tetra-sialylated glycoforms. Methods for producing variant sCR1 glycoforms comprising at least two sialylated glycans (e.g., di-, tri- or tetra-sialylated glycans), will be apparent to the skilled person and/or described herein.

Exemplary methods for determining the biological activity of the sCR1 variant of the disclosure will be apparent to the skilled person and/or described herein. For example, methods for determining inhibitory activity of the classical, lectin and/or alternative pathway are described herein.

### Conjugates

The present disclosure provides a sCR1 variant conjugate for use in any method described herein. Methods for conjugation of the sCR1 variant will be apparent to the skilled person and/or described herein. All forms and methods of conjugation (i.e., binding) are contemplated by the present disclosure, including, for example, direct conjugation between the sCR1 variant and another compound/moiety as described herein or indirect binding (e.g., by virtue of a linker between the sCR1 variant and the other compound/moiety). In one example, the conjugate is formed by a chemical conjugation (e.g., by an amine bond or disulphide bond) or by genetic fusion.

In one example, a sCR1 variant of the present disclosure is conjugated to a half-life extending moiety or a further soluble complement inhibitor.

In one example, the sCR1 variant of the present disclosure is conjugated to a half-life extending moiety or a further soluble complement inhibitor, which is directly or indirectly bound to the sCR1 variant. The half-life extending moiety or further soluble complement inhibitor can be directly or indirectly bound to the sCR1 variant (e.g., can comprise a linker in the case of indirect binding).

In one example, the sCR1 variant is conjugated to the half-life extending moiety or a further soluble complement inhibitor by an amine bond.

In one example, disclosure provides a fusion protein comprising the sCR1 variant and the half-life extending moiety or a further soluble complement inhibitor. For example, the half-life extending moiety or a further soluble complement inhibitor is positioned at N-terminus of the sCR1 variant, C-terminus of the sCR1 variant or any combination thereof.

In one example, the sCR1 variant is conjugated to the half-life extending moiety or a further soluble complement inhibitor via a linker. For example, the linker is a peptide linker.

In one example, the linker is a flexible linker. A "flexible" linker is an amino acid sequence which does not have a fixed structure (secondary or tertiary structure) in solution. Such a flexible linker is therefore free to adopt a variety of conformations. Flexible linkers suitable for use in the present disclosure are known in the art. An example of a flexible linker for use in the present invention is the linker sequence SGGGGS/GGGGS/GGGGS or (Gly4Ser)3. Flexible linkers are also disclosed in WO1999045132.

The linker may comprise any amino acid sequence that does not substantially hinder interaction of the binding region with its target. Preferred amino acid residues for flexible linker sequences include, but are not limited to, glycine, alanine, serine, threonine proline, lysine, arginine, glutamine and glutamic acid.

The linker sequences between the binding regions preferably comprise five or more amino acid residues. The flexible linker sequences according to the present disclosure consist of 5 or more residues, preferably, 5, 6, 7, 8, 9, 10, 11, 12, 13, 14, 15, 16, 17, 18, 19 or 20 or 25 or 30 or more residues. In a highly preferred embodiment of the invention, the flexible linker sequences consist of 5, 7, 10, 13 or 16 or 30 residues.

In one example, the flexible linker has an amino acid sequence according to SEQ ID NO: 31, i.e., GSGGSGGSGGSGS (GS13).

In one example, the flexible linker has an amino acid sequence according to SEQ ID NO: 35, i.e., SGGSGGSGGSGGSGGSGGSGGSGGSGGSGS (GS30).

Exemplary compounds that can be conjugated to a sCR1 variant of the disclosure and methods for such conjugation are known in the art and described herein.

### Half Life extending moieties

In one example, the sCR1 variant is conjugated to a half-life extending moiety. Half-life extending moieties suitable for use in the present disclosure will be apparent to the skilled person, and include, but are not limited to, those described herein. For example, the half-life extending moiety is selected from the group consisting of a human serum albumin or functional fragment thereof, an immunoglobulin Fc region or functional fragment thereof, afamin, alpha-fetoprotein, vitamin D binding protein, antibody fragments that bind to albumin and polymers.

In one example, the half-life extending moiety is a human serum albumin or functional fragment thereof.

In one example, the half-life extending moiety is an immunoglobulin Fc region or functional fragment thereof.

In one example, the half-life extending moiety is an afamin.

In one example, the half-life extending moiety is an alpha-fetoprotein.

In one example, the half-life extending moiety is a vitamin D binding protein.

In one example, the half-life extending moiety is an antibody fragment that binds to albumin.

In one example, the half-life extending moiety is a polymer.

### Albumin proteins and variants thereof

In one example, the half-life extending moiety is albumin, or a functional fragment or variant thereof.

Serum albumin, or blood albumin, is the most abundant blood protein and functions as a carrier protein for steroids, fatty acids and thyroid hormones in the blood, as well as playing a major role in stabilising extracellular fluid volume.

In one example, the albumin, functional fragment or variant thereof is serum albumin, such as human serum albumin. For the purposes of nomenclature only and not limitation an exemplary sequence of a mature human serum albumin is set out in NCBI GenBank Accession ID: AEE60908 and SEQ ID NO: 32.

In one example, the albumin, functional fragment or variant thereof, comprises one or more amino acid substitutions, deletions or insertions. Amino acid substitutions suitable for use in the present disclosure will be apparent to the skilled person and include naturally-occurring substitutions and engineered substitutions such as those described, for example, in WO2011051489, WO2014072481, WO2011103076, WO2012112188, WO2013075066, WO2015063611, WO2014179657 and WO2019075519.

In one example, the present disclosure provides a sCR1 variant conjugated to an albumin family protein, e.g., a protein that is structurally or evolutionarily related to albumin. For example, the sCR1 variant is conjugated to afamin, alpha-fetoprotein or a vitamin D binding protein.

In another example, the sCR1 variant is fused, e.g., expressed as a fusion protein, to an albumin family protein, e.g., a protein that is structurally or evolutionarily related to albumin. For example, the sCR1 variant is fused, e.g., as a fusion protein, to afamin, alpha-fetoprotein or a vitamin D binding protein.

### Immunoglobulin Fc regions and fragments thereof

In one example, the half-life extending moiety is an immunoglobulin or functional fragment thereof. For example, the immunoglobulin comprises an Fc region, such as an Fc domain or an Fc fragment and/or variant thereof. In one example, the Ig is a portion(s) of the immunoglobulin constant domain(s). For the purposes of nomenclature only and not limitation an exemplary sequence of a human IgG1 Fc is set out in SEQ ID NO: 33. For the purposes of nomenclature only and not limitation an exemplary sequence of a human IgG₄ Fc is set out in SEQ ID NO: 34.

In one example, the Fc fragment and/or variant thereof, comprises one or more amino acid substitutions, deletions or insertions. Amino acid substitutions suitable for use in the present disclosure will be apparent to the skilled person and include naturally-occurring substitutions and engineered substitutions such as those described, for example, in WO2000042072, WO2002060919, WO2004035752 and WO2006053301.

In one example, the immunoglobulin or fragment thereof for use in the present disclosure comprises IgG₄ constant regions or stabilized IgG₄ constant regions. For example, the stabilized IgG₄ constant regions comprise a proline at position 241 of the hinge region according to the system of Kabat (Kabat et al., Sequences of Proteins of Immunological Interest Washington DC United States Department of Health and Human Services, 1987 and/or 1991) or a proline at position 228 of the hinge region according to the EU numbering system (Edelman, G.M. et al., Proc. Natl. Acad. USA, 63, 78-85 (1969)).

In one example, the Fc domain is modified to prevent it being able to dimerize. For example, the Fc region is a monomeric Fc region.

Methods for generating half antibodies are known in the art and exemplary methods are described herein.

In one example, the half antibody can be secreted by introducing into cells genes of the protein that comprise the Fc domain of interest for expression. In one example, a constant region (e.g., an IgG₄ Fc domain) comprises a "key or hole" (or "knob or hole") mutation to prevent heterodimer formation. In one example, a constant region (e.g., an IgG₄ Fc domain) comprises a T366W mutation (or knob). In another example, a constant region (e.g., an IgG₄ Fc domain) comprises a T366S, L368A and Y407V mutation (or hole). In another example, the Fc domain comprises T350V, T366L, K392L and T394W mutations (knob). In another example, the constant region comprises T350V, L351Y, F405A and Y407V mutations (hole). Exemplary constant region amino acid substitutions are numbered according to the EU numbering system.

For example, the Fc domain is an IgG₄ Fc domain comprising the sequence set forth in SEQ ID NO: 34 with the following substitutions:
- An arginine substituted for the proline at position 228;
- A phenylalanine substituted for the leucine at position 351;
- An arginine substituted for the threonine at position 366;
- A lysine substituted for the proline at position 395;
- An arginine substituted for the phenylalanine at position 405; and
- A glutamic acid substituted for the tyrosine at position 407.

In one example, the present disclosure provides a sCR1 variant conjugated to an immunoglobulin or functional fragment thereof, e.g., an Fc region, such as an Fc domain or an Fc fragment and/or variant thereof. For example, the sCR1 variant is conjugated to human IgG₄ Fc.

In another example, the sCR1 variant is fused, e.g., expressed as a fusion protein, to an immunoglobulin or functional fragment thereof, e.g., an Fc region, such as an Fc domain or an Fc fragment and/or variant thereof. For example, the sCR1 variant is fused, e.g., as a fusion protein, to human IgG₄ Fc.

### Antibodies and fragments thereof

In one example, the immunoglobulin is an antibody or antigen binding fragment that binds to albumin. Exemplary antibodies or antigen binding fragments are known in the art and described, for example, in Kang et al, Immunol Lett.;169:33-40, 2016; Protein Eng Des Sel. 21(5):283-8, 2008; and Holt et al., MAbs. 8(7): 1336-1346, 2016.

Additional exemplary antibodies or antigen binding fragments thereof for use in the present disclosure are described herein or known in the art and include:
- a humanized antibody or fragment thereof, e.g., a protein comprising a human-like variable region, which includes CDRs from an antibody from a non-human species (e.g., mouse or rat or non-human primate) grafted onto or inserted into framework regions (FRs) from a human antibody (e.g., produced by methods described in US5225539, US6054297, US7566771 or US5585089)
- a human antibody or fragment thereof, e.g., antibodies having variable and, optionally, constant antibody regions found in humans, e.g. in the human germline or somatic cells or from libraries produced using such regions. The "human" antibodies can include amino acid residues not encoded by human sequences, e.g. mutations introduced by random or site directed mutations *in vitro* (e.g., produced by methods described in US5565332) and affinity matured forms of such antibodies.

- a synhumanized antibody or fragment thereof, e.g., an antibody that includes a variable region comprising FRs from a New World primate antibody variable region and CDRs from a non-New World primate antibody variable region (e.g., produced by methods described in WO2007019620).
- a primatized antibody or fragment thereof, e.g., an antibody comprising variable region(s) from an antibody generated following immunization of a non-human primate (e.g., a cynomolgus macaque) (e.g., produced by methods described in US6113898).
- a chimeric antibody or chimeric antigen binding fragment, e.g., an antibody or fragment in which one or more of the variable domains is from a particular species (e.g., murine, such as mouse or rat) or belonging to a particular antibody class or subclass, while the remainder of the antibody or fragment is from another species (such as, for example, human or non-human primate) or belonging to another antibody class or subclass (e.g., produced by methods described in US6331415; US5807715; US4816567 and US4816397).
- a deimmunized antibody or antigen binding fragment thereof, e.g., antibodies and fragments that have one or more epitopes, e.g., B cell epitopes or T cell epitopes removed (i.e., mutated) to thereby reduce the likelihood that a subject will raise an immune response against the antibody or protein (e.g., as described in WO2000034317 and WO2004108158).
- a bispecific antibody or fragment thereof, e.g., an antibody comprising two types of antibodies or antibody fragments (e.g., two half antibodies) having specificities for different antigens or epitopes (e.g., as described in US5731168).

Additional exemplary antibody fragments for use in the present disclosure are described herein or known in the art and include:
- single-domain antibodies (domain antibody or dAb), e.g., a single polypeptide chain comprising all or a portion of the heavy chain variable domain of an antibody.
- a diabody, triabody, tetrabody or higher order protein complex (e.g., as described in WO98/044001 and/or WO94/007921).
- single chain Fv (scFv) fragments, e.g., a fragment comprising V_{H} and V_{L} regions in a single polypeptide chain and a polypeptide linker between the V_{H} and V_{L} which enables the scFv to form the desired structure for antigen binding (i.e., for the V_{H} and V_{L} of the single polypeptide chain to associate with one another to form a Fv).
- a half-antibody or a half-molecule, e.g., a protein comprising a single heavy chain and a single light chain.

The present disclosure also contemplates other antibodies and antibody fragments, such as:
(i) minibodies, e.g., as described in US5837821;
(ii) heteroconjugate proteins, e.g., as described in US4676980;
(iii) heteroconjugate proteins produced using a chemical cross-linker, e.g., as described in US4676980; and
(iv) Fabs (e.g., as described in EP19930302894).

### Polymers

In one example, the present disclosure provides a sCR1 variant conjugated to a polymer. Suitable polymers for use in the present disclosure will be apparent to the skilled person and/or are described herein.

In one example, the sCR1 variant is conjugated to a polyethylene glycol (PEG). For example, the polymer comprises mono- or poly- (e.g., 2-4) polyethylene glycol (PEG) moieties. For example, the mono- poly- (e.g., 2-4) polyethylene glycol (PEG) moieties extend *in vivo* half-lives of the sCR1 variant.

Pegylation may be carried out by any of the pegylation reactions available. Exemplary methods for preparing pegylated protein products can generally include (a) reacting a polypeptide with polyethylene glycol (such as a reactive ester or aldehyde derivative of PEG) under conditions whereby the protein becomes attached to one or more PEG groups; and (b) obtaining the reaction product(s).

The skilled person will be aware of different PEG attachment methods which include, but are not limited to those described in e.g., EP 0 401 384; Malik et al., Exp. Hematol., 20:1028-1035 (1992); Francis, Focus on Growth Factors, 3(2):4-10 (1992); EP 0 154 316; EP 0 401 384; WO 92/16221; WO 95/34326; U.S. Pat. No. 5,252,714.

### Soluble complement inhibitors

The present disclosure provides a sCR1 variant conjugated to a further soluble complement inhibitor.

In one example, the sCR1 variant, is conjugated to a soluble complement inhibitor, or modified (i.e., variant) form thereof.

Suitable complement inhibitors for use in the present disclosure will be apparent to the skilled person and include, for example, Factor I, (fI), Factor H (fH), complement Factor H related protein (CFHR), C4b-binding protein (C4bp), soluble CD55 (decay accelerating factor (DAF)), soluble CD46 (membrane cofactor protein (MCP)), soluble CD59 (protectin), soluble complement receptor 2 (sCR2), TT30 (CR2-fH), Cobra venom factor (CVF) and a functional fragment or variant thereof.

### Assaying Activity of a sCR1 variant

sCR1 variants of the present disclosure are readily screened for biological activity, e.g., as described below.

### Measuring Complement Activity

In one example, complement activity is measured using an enzyme immunoassay (e.g., a Wieslab^{®} complement assay kit). For example, complement inhibitory activity is determined using labelled antibodies specific for an antigen or an epitope produced during complement activation (e.g., C5b-9 or an epitope present in C5b-9). In one example, the wells of a microtitre plate are coated with specific activators of the classical, lectin or alternative pathway. In another example, the sCR1 variant is incubated with normal human serum and appropriate assay diluent (i.e., a diluent comprising appropriate components to ensure specific activation of the classical, lectin or alternative pathway) and added to microtitre plate wells coated with specific activators of the classical, lectin or alternative pathway and the amount of C5b-9 complex formed is detected using a specific alkaline phosphatase labelled antibody to the C5b-9. In one example, the amount of complement activation product (i.e., C5b-9) produced is proportional to the functional activity of the complement pathway. In one example, the half maximal inhibitor concentration (i.e., IC₅₀) is determined. For example, the IC₅₀ of the sCR1 variant is determined and compared to the IC₅₀ of a sCR1 comprising a sequence set forth in SEQ ID NO: 2.

In another example, complement inhibitory activity is determined using a hemolysis assay (e.g., classical pathway (i.e., CH50) and alternative pathway (ApH50) inhibition assays). The CH50 assay is a method for measuring the total classical complement activity in serum. This test is a lytic assay, which uses antibody-sensitized erythrocytes as the activator of the classical complement pathway and human serum as complement source. The percent hemolysis can be determined, for example, using a spectrophotometer. The CH50 assay provides an indirect measure of terminal complement complex (TCC) formation, since the TCC themselves are directly responsible for the hemolysis that is measured. The assay is well known. Briefly, to assess the inhibition of the classical complement pathway, pre-diluted human serum is pre-incubated in microassay wells, together with serially diluted sCR1 variants. Next, antibody-sensitized erythrocytes (e.g., sheep erythrocytes sensitized with rabbit anti-sheep antibodies) are added. After centrifugation, free haemoglobin is measured in the supernatants, using a spectrophotometer. The decrease in free haemoglobin reflects the inhibition of TCC-mediated erythrocyte lysis. sCR1-mediated inhibition is then calculated relative to erythrocytes which were incubated with human serum only (100 % lysis sample). Complement inhibition can also be evaluated based on any methods known in the art, including for example, in vitro zymosan assays, assays for lysis of erythrocytes, antibody or immune complex activation assays, alternative pathway activation assays, and lectin pathway activation assays.

### Pharmaceutical Compositions and Methods of Treatment

Suitably, in compositions or methods for administration of the sCR1 variant of the disclosure to a subject, the sCR1 variant conjugate of the present disclosure (i.e., the sCR1 variant conjugated to a half-life extending moiety or further soluble complement inhibitor) is combined with a pharmaceutically acceptable carrier as is understood in the art. Accordingly, one example of the present disclosure provides a composition (e.g., a pharmaceutical composition) comprising the sCR1 variant of the disclosure combined with a pharmaceutically acceptable carrier. A further example of the present disclosure provides a composition (e.g., a pharmaceutical composition) comprising the sCR1 variant conjugate of the disclosure combined with a pharmaceutically acceptable carrier.

In general terms, by "carrier" is meant a solid or liquid filler, binder, diluent, encapsulating substance, emulsifier, wetting agent, solvent, suspending agent, coating or lubricant that may be safely administered to any subject, e.g., a human. Depending upon the particular route of administration, a variety of acceptable carriers, known in the art may be used, as for example described in Remington's Pharmaceutical Sciences (Mack Publishing Co. N.J. USA, 1991). A sCR1 variant or sCR1 variant conjugate of the present disclosure is useful for parenteral, topical, oral, or local administration, aerosol administration, intrathecal administration or transdermal administration, for prophylactic or for therapeutic treatment. In one example, the sCR1 variant or sCR1 variant conjugate is administered parenterally, such as subcutaneously or intravenously. For example, the sCR1 variant or sCR1 variant conjugate is administered intravenously.

Formulation of a sCR1 variant to sCR1 variant conjugate to be administered will vary according to the route of administration and formulation (e.g., solution, emulsion, capsule) selected. An appropriate pharmaceutical composition to be administered can be prepared in a physiologically acceptable carrier. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. A variety of appropriate aqueous carriers are known to the skilled artisan, including water, buffered water, buffered saline, polyols (e.g., glycerol, propylene glycol, liquid polyethylene glycol), dextrose solution and an amino acid, including for example, glycine, proline, lysine, histidine, methionine, arginine, alanine, valine, serine, asparagine, phenylalanine, tyrosine, cysteine, threonine, leucine, tryptophan, glutamine, isoleucine, glutamate and combinations thereof. Intravenous vehicles can include various additives, preservatives, or fluid, nutrient or electrolyte replenishers (See, generally, Remington's Pharmaceutical Science, 16th Edition, Mack, Ed. 1980). The compositions can optionally contain pharmaceutically acceptable auxiliary substances as required to approximate physiological conditions such as pH adjusting and buffering agents and toxicity adjusting agents, for example, sodium acetate, sodium chloride, potassium chloride, calcium chloride and sodium lactate. The composition can be stored in the liquid stage or can be lyophilized for storage and reconstituted in a suitable carrier prior to use according to art-known lyophilization and reconstitution techniques.

A method of the present disclosure may also include co-administration of the sCR1 variant or sCR1 variant conjugate according to the disclosure together with the administration of another therapeutically effective agent for inhibiting complement activity or for the prevention or treatment of a complement mediated disorder.

In one example, the sCR1 variant or conjugate thereof of the disclosure is used in combination with at least one additional known compound or therapeutic protein which is currently being used or is in development for inhibiting complement activity or preventing or treating complement mediated disorders. Compounds currently used in the treatment of complement mediated disorders are known in the art, and include antibodies against C5 and activated forms thereof (C5a), e.g., eculizumab, Berinert Human C1 esterase inhibitor, Human C1 esterase inhibitor, Ruconest Recombinant C1 esterase inhibitor, Cinryze Human C1 esterase inhibitor, Anti human MASP-2 monoclonal antibody, APL-2 C3-inhibiting peptide, Lampalizumab, TNT009 Anti-C1s Antibody. Additional compounds are described in Reis et al., Clin Immunol. Dec; 161(2): 225-240, 2015.

As will be apparent from the foregoing, the present disclosure provides methods of concomitant therapeutic treatment of a subject, comprising administering to a subject in need thereof an effective amount of a first agent and a second agent, wherein the first agent is a sCR1 variant or sCR1 variant conjugate of the present disclosure, and the second agent is also for inhibiting complement activity or for the prevention or treatment of a complement mediated disorder.

As used herein, the term "concomitant" as in the phrase "concomitant therapeutic treatment" includes administering a first agent in the presence of a second agent. A concomitant therapeutic treatment method includes methods in which the first, second, third or additional agents are co-administered. A concomitant therapeutic treatment method also includes methods in which the first or additional agents are administered in the presence of a second or additional agent, wherein the second or additional agent, for example, may have been previously administered. A concomitant therapeutic treatment may be executed step-wise by different actors. For example, one actor may administer to a subject a first agent and as a second actor may administer to the subject a second agent and the administering steps may be executed at the same time, or nearly the same time, or at distant times, so long as the first agent (and/or additional agents) are after administration in the presence of the second agent (and/or additional agents). The actor and the subject may be the same entity (e.g. a human).

The optimum concentration of the active ingredient(s) in the chosen medium can be determined empirically, according to procedures known to the skilled artisan, and will depend on the ultimate pharmaceutical formulation desired.

The dosage ranges for the administration of the sCR1 variant of the disclosure are those large enough to produce the desired effect. For example, the composition comprises an effective amount of the sCR1 variant or sCR1 variant conjugate. In one example, the composition comprises a therapeutically effective amount of the sCR1 variant or sCR1 variant conjugate. In another example, the composition comprises a prophylactically effective amount of the sCR1 variant or sCR1 variant conjugate.

The dosage should not be so large as to cause adverse side effects. Generally, the dosage will vary with the age, condition, sex and extent of the disease in the patient and can be determined by one of skill in the art. The dosage can be adjusted by the individual physician in the event of any complication.

Dosage can vary from about 0.1 mg/kg to about 300 mg/kg, e.g., from about 0.2 mg/kg to about 200 mg/kg, such as, from about 0.5 mg/kg to about 20 mg/kg, in one or more dose administrations daily, for one or several days.

In some examples, the sCR1 variant or sCR1 variant conjugate is administered at an initial (or loading) dose which is higher than subsequent (maintenance doses). For example, the sCR1 variant or sCR1 variant conjugate is administered at an initial dose of between about 10mg/kg to about 30mg/kg. The sCR1 variant or sCR1 variant conjugate is then administered at a maintenance dose of between about 0.0001mg/kg to about 30mg/kg. The maintenance doses may be administered every 2-30 days, such as, every 2 or 3 or 6 or 9 or 12 or 15 or 18 or 21 or 24 or 27 or 30 days.

In some examples, a dose escalation regime is used, in which a sCR1 variant or sCR1 variant conjugate is initially administered at a lower dose than used in subsequent doses. This dosage regime is useful in the case of subject's initially suffering adverse events.

In the case of a subject that is not adequately responding to treatment, multiple doses in a week may be administered. Alternatively, or in addition, increasing doses may be administered.

A subject may be retreated with the sCR1 variant or sCR1 variant conjugate, by being given more than one exposure or set of doses, such as at least about two exposures, for example, from about 2 to 60 exposures, and more particularly about 2 to 40 exposures, most particularly, about 2 to 20 exposures.

In one example, any retreatment may be given when signs or symptoms of disease return, e.g., a bacterial infection.

In another example, any retreatment may be given at defined intervals. For example, subsequent exposures may be administered at various intervals, such as, for example, about 24-28 weeks or 48-56 weeks or longer. For example, such exposures are administered at intervals each of about 24-26 weeks or about 38-42 weeks, or about 50-54 weeks.

In the case of a subject that is not adequately responding to treatment, multiple doses in a week may be administered. Alternatively, or in addition, increasing doses may be administered.

In another example, for subjects experiencing an adverse reaction, the initial (or loading) dose may be split over numerous days in one week or over numerous consecutive days.

Administration of a sCR1 variant or sCR1 variant conjugate according to the methods of the present disclosure can be continuous or intermittent, depending, for example, on the recipient's physiological condition, whether the purpose of the administration is therapeutic or prophylactic, and other factors known to skilled practitioners. The administration may be essentially continuous over a preselected period of time or may be in a series of spaced doses, e.g., either during or after development of a condition.

### Kits and Other Compositions of Matter

Another example of the disclosure provides kits containing a sCR1 variant or sCR1 variant conjugate of the present disclosure useful for inhibiting complement activity or for the treatment or prevention of a complement mediated disorder as described above.

In one example, the kit comprises (a) a container comprising a sCR1 variant or sCR1 variant conjugate optionally in a pharmaceutically acceptable carrier or diluent; and (b) a package insert with instructions for inhibiting complement activity or for treating or preventing a complement mediated disorder in a subject.

In one example, the kit comprises (a) at least one sCR1 variant or sCR1 variant conjugate optionally in a pharmaceutically acceptable carrier or diluent; (b) instructions for using the kit in inhibiting complement activity or for treating or preventing a complement mediated disorder in the subject; and (c) optionally, at least one further therapeutically active compound or drug.

In accordance with this example of the disclosure, the package insert is on or associated with the container. Suitable containers include, for example, bottles, vials, syringes, etc. The containers may be formed from a variety of materials such as glass or plastic. The container holds or contains a composition that is effective for inhibiting complement activity or for treating or preventing a complement mediated disorder and may have a sterile access port (for example, the container may be an intravenous solution bag or a vial having a stopper pierceable by a hypodermic injection needle). At least one active agent in the composition is the sCR1 variant. The label or package insert indicates that the composition is used for treating a subject eligible for treatment, e.g., one having or predisposed to developing a complement mediated disorder, with specific guidance regarding dosing amounts and intervals of the sCR1 variant and any other medicament being provided. The kit may further comprise an additional container comprising a pharmaceutically acceptable diluent buffer, such as bacteriostatic water for injection (BWFI), phosphate-buffered saline, Ringer's solution, and/or dextrose solution. The kit may further include other materials desirable from a commercial and user standpoint, including other buffers, diluents, filters, needles, and syringes.

The kit optionally further comprises a container comprising a second medicament, wherein the sCR1 variant or sCR1 variant conjugate is a first medicament, and which article further comprises instructions on the package insert for treating the subject with the second medicament, in an effective amount. The second medicament may be a therapeutic protein set forth above.

The present disclosure includes the following non-limiting Examples.

### EXAMPLES

### Example 1: Generation of sCR1 variants

Human Complement Receptor Type 1 (CR1) cDNA (GenBank Accession no. NP_000564) was codon-optimized for human expression and synthesized by Geneart^{®} (Invitrogen^{™}, Thermo Fisher Scientific). Full-length and truncated soluble CR1 (sCR1) variants were generated using standard PCR-based mutagenesis techniques. cDNA was generated with a Kozak consensus sequence (GCCACC) immediately upstream of the initiating methionine (+1), following which it was digested with NheI and XhoI and ligated into pcDNA3.1 (Invitrogen^{™}, Thermo Fisher Scientific). sCR1 variant cDNA was cloned in-frame with a C-terminal 8x Histidine-tag. See Table 1 for a list of sCR1-8His variants.

Large-scale preparations of plasmid DNA were carried out using QIAGEN Plasmid Giga Kits according to the manufacturer's instructions. The nucleotide sequences of all plasmid constructs were verified by sequencing both strands using BigDye^{™} Terminator Version 3.1 Ready Reaction Cycle Sequencing (Invitrogen^{™}, Thermo Fisher Scientific) and an Applied Biosystems 3130xl Genetic Analyzer.

Transient transfections of Expi293F^{™} cells with sCR1 expression plasmids were performed using the Expi293^{™} Expression system according to the manufacturer's recommendations (Invitrogen^{™}, Thermo Fisher Scientific). All cell culture media were supplemented with Antibiotic-Antimycotic (GIBCO^{®}, Thermo Fisher Scientific) and cells were maintained at 37°C in incubators with an atmosphere of 8% CO₂.

sCR1-8His polypeptides were purified. Briefly, for purification of hexahistidine tagged sCR1 proteins, the culture supernatant was loaded directly onto nickel sepharose excel affinity resin (GE Healthcare) pre-equilibrated with 20mM NaH₂PO₄, 500mM NaCl, 10mM Imidazole, pH 7.4. After loading, the resin was washed with 20mM NaH₂PO₄, 500mM NaCl, 25mM Imidazole, pH 7.4. Resin-bounded sCR1 was block eluted with 20mM NaH₂PO₄, 500mM NaCl, 500mM Imidazole, pH 7.4 collecting eluted protein based on absorbance at 280nm. Collected protein was loaded onto a HiLoad 26/60 superdex200 prep grade column (GE Healthcare) pre-equilibrated in mt-PBS (137mM NaCl, 27mM KCl, 8.1mM Na₂HPO₄, 1.15mM KH₂PO₄, pH 7.4) to remove any contaminating proteins and buffer exchange into desired buffer. Purified protein was concentrated using amicon ultra centrifugal filters with 50kDa MWCO to desired concentration, sterile filtered and stored at -80°C. Due to intracellular processing, the mature sCR1-8His variants lack the N-terminal 41 aa human CR1 signal peptide.

**Table 1: sCR1-8His variants**

| **Identifier** | **LHR regions** | **Length of mature sCR1 variant protein (aa's) - (less 41 aa human CR1 signal peptide and less 8His tag)** | **SEQ ID NO:** |
|---|---|---|---|
| sCR1(1971)-8His SEQ ID NO: 20 | ABCD | 1930 | Signal peptide: SEQ ID NO: 18 |
| | | | sCR1 sequence: SEQ ID NO: 2 |
| | | | 8xHis-tag: SEQ ID NO: 17 |
| sCR1(1392)-8His SEQ ID NO: 21 | ABC | 1351 | Signal peptide: SEQ ID NO: 18 |
| | | | sCR1 sequence: SEQ ID NO: 3 |
| | | | 8xHis-tag: SEQ ID NO: 17 |
| sCR1(939)-8His SEQ ID NO: 22 | AB | 898 | Signal peptide: SEQ ID NO: 18 |
| | | | sCR1 sequence: SEQ ID NO: 4 |
| | | | 8xHis-tag: SEQ ID NO: 17 |
| sCR1(490-1392)-8His SEQ ID NO: 23 | BC | 903 | Signal peptide: SEQ ID NO: 19 |
| | | | sCR1 sequence: SEQ ID NO: 5 |
| | | | 8xHis-tag: SEQ ID NO: 17 |
| sCR1(490-1971)-8His SEQ ID NO: 24 | BCD | 1482 | Signal peptide: SEQ ID NO: 19 |
| | | | sCR1 sequence: SEQ ID NO: 6 |
| | | | 8xHis-tag: SEQ ID NO: 17 |
| sCR1(234)-8His SEQ ID NO: 25 | A' | 193 | Signal peptide: SEQ ID NO: 18 |
| | | | sCR1 sequence: SEQ ID NO: 7 |
| | | | 8xHis-tag: SEQ ID NO: 17 |
| sCR1(489)-8His SEQ ID NO: 26 | A | 448 | Signal peptide: SEQ ID NO: 18 |
| | | | sCR1 sequence: SEQ ID NO: 8 |
| | | | 8xHis-tag: SEQ ID NO: 17 |
| sCR1(940-1971)-8His SEQ ID NO: 27 | CD | 1032 | Signal peptide: SEQ ID NO: 19 |
| | | | sCR1 sequence: SEQ ID NO: 9 |
| | | | 8xHis-tag: SEQ ID NO: 17 |
| sCR1(490-939)-8His SEQ ID NO: 28 | B | 450 | Signal peptide: SEQ ID NO: 19 |
| | | | sCR1 sequence: SEQ ID NO: 10 |
| | | | 8xHis-tag: SEQ ID NO: 17 |
| sCR1(940-1392)-8His SEQ ID NO: 29 | C | 453 | Signal peptide: SEQ ID NO: 19 |
| | | | sCR1 sequence: SEQ ID NO: 11 |
| | | | 8xHis-tag: SEQ ID NO: 17 |
| sCR1(1393-1971)-8His SEQ ID NO: 30 | D | 579 | Signal peptide: SEQ ID NO: 19 |
| | | | sCR1 sequence: SEQ ID NO: 12 |
| | | | 8xHis-tag: SEQ ID NO: 17 |

### Example 2: sCR1-8His variants have complement inhibitory activity in vitro

To assess complement inhibitory activity, the sCR1-8His variants were tested in the Wieslab^{®} complement assay (Euro Diagnostica) according to manufacturer's instructions. Briefly, sCR1-8His variant proteins were serially diluted in PBS in a 96-well plate. 50 µl of each diluted sCR1-8His variant sample or PBS alone was added to 202.5 µl of pre-diluted human serum (1:101 for classical/lectin) or 220 µl of diluted serum (1:18 for alternative) in the appropriate assay diluent for each complement pathway (as per manufacturer's instructions) and incubated for 30 min at room temperature (RT). Once added to the pre-diluted serum, the final starting concentration of each protein was 40 nM. 100 µl of each sample was transferred to the assay plate in duplicate and incubated for 1 hr at 37°C (with no CO₂). Wells were emptied and washed three times with 300 µl/well of 1 × wash buffer (as per manufacturer's instructions). The terminal complex of C5b-9 was detected using 100 µl/well alkaline-phosphatase conjugated anti-C5b-9 specific monoclonal antibody, which was incubated for 30 min at RT. Unbound antibody was discarded and wells were washed three times with 300 µl/well of 1 × wash buffer. Bound antibodies were detected using 100 µl/well alkaline phosphatase substrate solution and incubated for 30 min at RT. Absorbance at 405 nm was read using the Envision plate reader.

Raw values were expressed as a percentage of C5b-9 formation by the serum and PBS only control (i.e. 100% C5b-9 formation). Results were analysed in Graph Pad Prism for IC₅₀ values using a log(inhibitor) vs. response -- Variable slope (four parameters) fit. Bottom and top constrained to values 0 and 100, respectively.

All sCR1-8His variants except sCR1(490-939)-8His, sCR1(940-1392)-8His and sCR1(1393-1971)-8His had functional activity in the classical, lectin and alternative pathways. sCR1(490-939)-8His had functional activity in the alternative pathway only, whilst sCR1(940-1392)-8His had functional activity in the lectin and alternative pathways. sCR1(1939-1971)-8His had no detectable activity in any of the classical, lectin or alternative pathways.

As shown in **Table 2** below, sCR1(1392)-8His had increased inhibitory activity in all three complement pathways (i.e., classical, lectin and alternative) compared to full-length sCR1(1971)-8His and other sCR1 fragments in the Wieslab assays.

sCR1-8His variants were also tested for functional activity using a hemolysis assay (e.g., classical pathway (i.e., CH50) and alternative pathway (ApH50) inhibition assays).

To assess the inhibition of the classical pathway of the complement system (i.e., CH50) by sCR1 variants, sheep erythrocytes (Siemens) were sensitized with rabbit anti-sheep antibodies (Ambozeptor 6000; Siemens) and diluted to 4×10⁸ cells/mL GVB⁺⁺ (GVB, 0.15 mM CaCl₂, 0.5 mM MgCl₂). sCR1 variants were pre-incubated in 1/40 diluted NHS (30 min at RT) and subsequently added to the erythrocytes at a 1/1 (v/v) ratio and incubated during 1 h at 37°C in a microtiter-plate shaking device. After adding ice-cold GVBE (GVB, 10 mM EDTA) and centrifugation (5 min at 1250 × g, 4°C), hemolysis was determined in the supernatant by measuring the absorbance of released hemoglobin at 412 nm. Cells incubated with NHS and buffer only served as 100 % lysis controls. The inhibition of lysis by the sCR1 variants was calculated relative to control.

To assess the inhibition of the alternative pathway of the complement system (i.e., ApH50) by sCR1 variants, rabbit erythrocytes (Jackson Laboratories) were washed and diluted to 2×10⁸ cells/mL GVB/MgEGTA (GVB, 5 mM MgEGTA). sCR1 variants were pre-incubated in 1/6 diluted NHS (30 min at RT) and subsequently added to the erythrocytes at a 2/1 (v/v) ratio and incubated during 1 h at 37°C in a microtiter-plate shaking device. After adding ice-cold GVBE and centrifugation (10 min at 1250 × g), hemolysis was determined in the supernatant by measuring the absorbance of released hemoglobin at 412 nm. Cells incubated with NHS and buffer only served as 100 % lysis controls. The inhibition of lysis by the sCR1 variants was calculated relative to control.

All variants except sCR1(1393-1971)-8His displayed functional activity in both the CH50 and ApH50 assays. As shown in **Table 3** sCR1(1392)-8His had increased activity compared to sCR1(1971)-8His in both assays.

**Table 2: Relative in vitro activity of sCR1 variants in Wieslab assays**

| **sCR1 variant** | **Wieslab Assay** | | | |
|---|---|---|---|---|
| | **Exp't no.** | **Classical IC₅₀ (nM)** | **Lectin IC₅₀ (nM)** | **Alternative IC₅₀ (nM)** |
| sCR1(1971)-8His [ABCD] | 1 | 1.50 | 1.20 | 0.821 |
| | 2 | 1.18 | 0.66 | 0.876 |
| | 3 | 1.40 | 0.78 | - |
| sCR1(1392)-8His [ABC] | 1 | 0.879 | 0.547 | 0.272 |
| | 2 | 0.402 | 0.428 | 0.384 |
| | 3 | 0.583 | 0.458 | - |
| sCR1(939)-8His [AB] | 1 | 4.04 | 2.20 | 3.41 |
| | 2 | 1.60 | 1.01 | 2.72 |
| sCR1(490-1971) [BCD] | 1 | 7.02 | 3.80 | 0.295 |
| | 2 | 2.90 | 4.29 | 1.31 |
| sCR1(490-1392) [BC] | 1 | 12.95 | 5.08 | 0.579 |
| | 2 | 5.70 | 2.33 | 1.33 |
| sCR1(1-234) [A'] | | 23.68 | 15.4 | 1.31 |
| sCR1(1-489) [A] | | 45.26 | 27.8 | 2.73 |
| sCR1(940-1971) [CD] | | 180.4 | 121.6 | 2.58 |
| CR1(490-939) [B] | | No Activity | ND | 3.21 |
| CR1(940-1392) [C] | | No Activity | 898.2 | 1.45 |
| CR1(1393-1971) [D] | | No Activity | No Activity | No Activity |

**Table 3: Relative in vitro activity of sCR1 variants in hemolysis assays**

| **sCR1 variant** | **Hemolysis Assay** | |
|---|---|---|
| | **Classical IC₅₀ (pM)** | **Alternative IC₅₀ (pM)** |
| sCR1(1971)-8His [ABCD] | 1.03 | 2.11 |
| sCR1(1392)-8His [ABC] | 0.427 | 0.956 |
| sCR1(939)-8His [AB] | 4.44 | 13.35 |
| sCR1(490-1971) [BCD] | 32.05 | 45.46 |
| sCR1(490-1392) [BC] | 9.69 | 13.88 |
| sCR1(1-234) [A'] | 35.7 | 45.09 |
| sCR1(1-489) [A] | 74.95 | 51.59 |
| sCR1(940-1971) [CD] | 658.5 | 90.49 |
| sCR1(490-939) [B] | 948.5 | 64.38 |
| sCR1(940-1392) [C] | 716.4 | 63.62 |
| sCR1(1393-1971) [D] | No Activity | No Activity |

### Example 3: sCR1(1392)-8His variant shows increased stability compared to sCR1(1971)-8His

To assess the stability of sCR1(1392)-8His in different buffer conditions, a differential scanning fluorimetry (DSF) assay was performed to measure the thermal stability of the sCR1(1392)-8His protein compared to the full length sCR1(1971)-8His protein. The stability of the proteins was assessed under a range of salt (NaCl 0mM, 50mM, 150mM and 500mM) and pH conditions for the following buffers: citrate, HEPES, sodium acetate, phosphate, glycine, histidine, TRIS and proline.

Briefly, 5 µl of 4x buffer concentrate were dispensed in duplicate in a 384-well plate. sCR1(1392)-8His and sCR1(1971)-8His proteins were diluted to 0.13 mg/ml in MT-PBS then spiked with a 1/20 dye stock (Sypro^{®} Orange; Sigma) made up in water to give a 1/400 final dilution in each assay reaction. 15 µl of protein/dye mixture were then dispensed into each well of the 384-well plate containing the buffer concentrate. The plate was sealed with an optical adhesive cover and centrifuged for 1 minute at 3220g prior to running on the QuantStudio^{™} Real-Time PCR instrument (Applied Biosystems). A melt curve was generated by cooling and holding the temperature for 1 minute at 20.0°C, before ramping up from 20.0°C to 99.0°C at a rate of 0.05°C /s. Protein Thermal Shift software (Applied Biosystems) was used to calculate the transition midpoint (Tₘ) values from each melting curve using the first derivative function. Contour plots were generated using JMP13 to graphically display how the Tₘ values change in relation to NaCl concentration (x axis) and pH (y axis).

sCR1(1392)-8His was stable under several buffer conditions including: phosphate (pH6.0-8.0; NaCl 0-500mM); phosphate-citrate (pH6.0-8.0; NaCl 0-500mM); Tris (pH7.0-9.0; NaCl 0-500mM); glycine (pH9.0-10.0; NaCl 0-500mM); HEPES (pH6.5-8.5; NaCl 0-500mM) and histidine (pH6.0-7.0; NaCl 0-500mM). The maximum Tₘ value measured was 61.4°C for sCR1(1392)-8His and 61.7°C for sCR1(1971)-8His.

Based on the buffer screen, sCR1(1392)-8His was more stable than sCR1(1971)-8His.

### Example 4: Sialylated sCR1(1392)-8His has improved in vivo half-life

To assess whether the *in vivo* half-life of sCR1(1392)-8His could be extended, a sialylated version of sCR1(1392)-8His was prepared (sCR1(1392)-8His^{SIA}). Briefly, the sialylated material was generated by co-transfecting Expi293F cells with the cDNA encoding sCR1(1392)-8His together with the cDNA encoding human ST3GAL3 (ST3 beta-galactoside alpha-2,3-sialyltransferase 3, GenBank Accession no. NP_006270) and the cDNA encoding human B4GALT1 (human β1,4-galactosyltransferase, GenBank Accession no. NP_001488.2) at a 94:3:3 ratio.

As shown in **Table 4,** sCR1(1392)-8His^{SIA} material produced in ST3GAL3/B4GALT1-transfected cells had a much higher proportion of sialylated glycans. In particular, sialylated sCR1(1392)-8His^{SIA} material had a higher proportion of di-, tri- and tetra-sialylated glycans.

**Table 4: Proportion of glycans in sialylated sCR1(1392)-8His**

| | | **sCR1(1392)-8His** | **sCR1(1392)-8His^{SIA}** |
|---|---|---|---|
| **Peak No.** | **Glycan Group** | **% of Total Peak Area** | **% of Total Peak Area** |
| 1 | Asialylated | 74.5 | 24.1 |
| 2 | Monosialvlated | 21.1 | 22.8 |
| 3 | Disialylated | 3.7 | 41.9 |
| 4 | Trisialylated | 0.6 | 9.1 |
| 5 | Tetrasialylated | 0.1 | 2.1 |

The *in vivo* half-life of sCR1(1392)-8His and the sialylated version thereof (sCR1(1392)-8His^{SIA}) was tested in human FcRn transgenic mice (B6.Cg-*Fcgrt^{tm1Dcr}* Tg(FCGRT)32Dcr/DcrJ; The Jackson Laboratory stock number 014565). Mice were intravenously (a single bolus injection into the tail vein) injected with 30mg/kg of sCR1(1392)-8His or sCR1(1392)-8His^{SIA} and plasma collected at various time points (Group A: 5min and 4h, n = 3; Group B: 0.5h and 8h, n = 3; Group C: 1h and 16h, n = 3; Group D: 2h and 48h, n =3). Blood was mixed with citrate buffer at a ratio of 8 parts blood 2 parts citrate buffer. Plasma levels of human sCR1 were measured in an anti-human CD35 ELISA (RayBiotech, cat no. ELH CD35) according to manufacturer's instructions, with the following modifications: standard curves (ranging from 3-250ng/mL) were generated using each test article, the assay buffer used was 1% BSA heat shock fraction, protease free (Sigma cat no. A3059), and the wash buffer was PBS + 0.05% v/v Tween-20. Mean residence time (MRT) and the area under the curve (AUC) were calculated using standard statistical formulae.

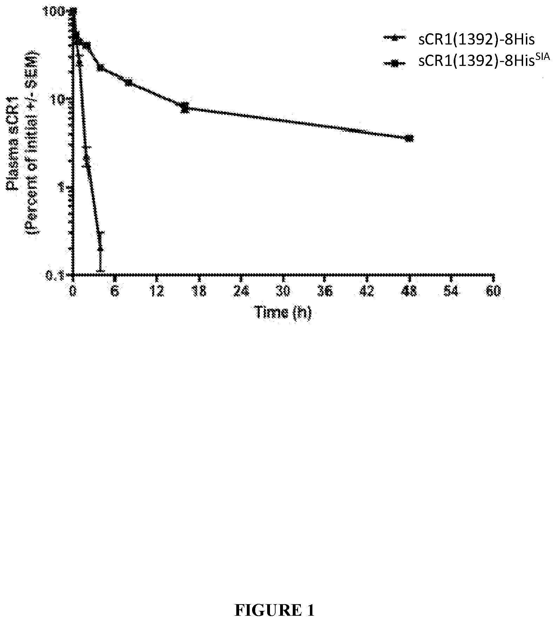

As shown in **Figure 1****,** the sCR1(1392)-8His^{SIA} had improved *in vivo* retention compared to sCR1(1392)-8His, with a 25-fold increased MRT (14.7 hours vs 35 mins) and an 8-fold increase in the AUC (AUC=516.5 vs 65.74).

### Example 5: sCR1(1392)-8His reduces anti-GBM glomerulonephritis

The effect of sCR1(1392)-8His treatment was assessed in an *in vivo* model of anti-glomerular basement membrane (GBM) glomerulonephritis. Briefly, anti-GBM glomerulonephritis was induced in C57BL/6 mice by intravenously injecting 1 mg of polyclonal rabbit anti-GBM antiserum (IgG fraction) at day 0, followed by intraperitoneal injection on day 6 with 2 mg of the mouse monoclonal anti-rabbit IgG (MsαRb IgG produced from hybridoma CRL-1753 (ATCC)). Mice were intraperitoneally injected with PBS or 60 mg/kg of sCR1(1392)-8His, sCR1(1971)-8His, or anti-mouse C5 mAb (muBB5.1-mIgG1 □□ Rother R, et al. Nat Biotechnol. 2007; Wang Y et al., Proc Natl Acad Sci 1995) on days 5 and 6. An additional group of mice was treated with 60 mg/kg sCR1(1392)-8His on day 6 only and not on day 5 (sCR1(1392)-8His x1). . On day 6, the drug (i.e. sCR1 variant or BB5.1) or PBS control was administered approximately one hour before the injection with the MsαRb IgG mAb, After the injection of MsαRb, mice were placed individually in metabolic cages to collect urine over a period of 24 hours. Urine albumin levels were measured with an ELISA kit (Bethyl Laboratories) and albuminuria per mouse is plotted as µg/24 h.

The sCR1(1392)-8His and sCR1(1971)-8His material used in this experiment was relatively unsialylated and was produced in Expi293F cells that were not co-transfected with ST3GAL3 and B4GALT1 cDNA.

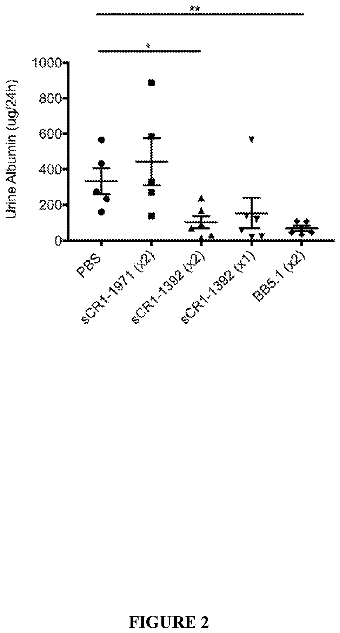

As shown in **Figure 2****,** urine albumin levels were significantly reduced in sCR1(1392)-8His-treated mice (PBS vs sCR1(1392)-8His x2: p=0.0147; PBS vs sCR1(1392)-8His x1: p=0.1526; PBS vs BB5.1: p=0.0078).

### Example 6: Sialylated sCR1(1392)-8His reduces anti-GBM glomerulonephritis

The effect of sCR1(1392)-8His^{SIA} treatment was assessed in an *in vivo* model of anti-glomerular basement membrane (GBM) glomerulonephritis, as previously described in Example 4. Mice were intraperitoneally injected with PBS or either 10mg/kg, 30mg/kg or 60 mg/kg of sCR1(1392)-8His^{SIA} on day 6.

On day 6, sCR1(1392)-8His^{SIA} or PBS control was administered approximately one hour before injection with the MsαRb IgG mAb, After the injection of MsαRb, mice were placed individually in metabolic cages to collect urine over a period of 24 hours. Urine albumin levels were measured with an ELISA kit (Bethyl Laboratories) and albuminuria per mouse is plotted as µg/24 h.

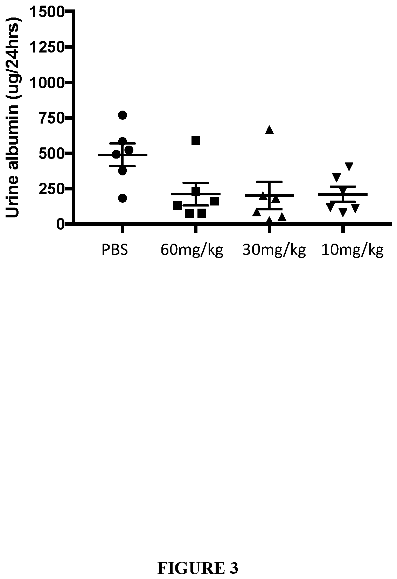

As shown in **Figure 3****,** urine albumin levels were reduced in mice treated with 10mg/kg, 30mg/kg and 60mg/kg sCR1(1392)-8His^{SIA}-treated mice compared to control (i.e., PBS) treated mice.

### Example 7: Sialylated sCR1(1392)-8His protects against renal ischemia-reperfusion injury

The effect of sCR1(1392)-8His^{SIA} treatment was assessed in an *in vivo* model of warm renal ischemic-reperfusion (IR) injury. Male 10-20 week old C57BL/6 mice were anesthetized and subjected to right nephrectomy and 22 minutes left renal ischemia, or right nephrectomy only (Sham), at 37°C. Briefly, a midline abdominal incision was made and the renal pedicles were bluntly dissected. After right nephrectomy, a microvascular clamp was placed on the left renal pedicle for 22min, while the animal was kept at 37°C. The clamp was removed after ischemia and the kidney observed to confirm complete reperfusion. Mice were treated with i.p. administration of 60 mg/kg sCR1(1392)-8His^{SIA} (n=14), or vehicle control (n=8), 1 hr prior to ischemia. Mice were sacrificed 24 hrs after reperfusion, and serum and plasma were collected to assess renal function (creatinine, urea) and complement activation (C3b, C5a ELISA). Kidneys were harvested to analyse complement C9 deposition and immune cell infiltration by immunofluorescence/confocal microscopy.

As shown in **Table 5** below, compared to Sham, severe renal injury was induced following IR in the vehicle-treated mice as indicated by significantly increased serum creatinine and urea, plasma C3b and C5a, and tissue C9 deposition, and neutrophil and macrophage infiltration. Treatment with sCR1(1392)-8His^{SIA} significantly protected against IR-induced damage, manifested by significantly lowered renal dysfunction (i.e., serum creatinine, urea), complement activation and deposition (i.e., plasma C3b, C5a, and tissue C9 deposition), and cellular infiltration (i.e., neutrophil and macrophage infiltration) (see **Table 5**)**.**

The results showed that sCR1(1392)-8His^{SIA} protected against IR-mediated renal damage in this model and was associated with markedly reduced loss of renal function indicated by serum creatinine and urea, as well as lowered plasma complement activation products, and tissue deposition of complement and infiltration by innate immune cells.

**Table 5: The effect of sCR1(1392)-8His^{SIA} on IR-mediated renal damage**

| | Sham | Vehicle | sCR1(1392)-8His^{SIA} | p value (Vehicle vs. sCR1 (1392)-8His^{SIA}) |
|---|---|---|---|---|
| Creatinine (µM) | 18.5±1.1 | 181.1±36.2 | 64.9±72.4 | 0.003 |
| Urea (mg/dL) | 55.5±6.3 | 384.8±52.5 | 142.4±145.1 | 0.02 |
| Plasma C3b (AU/ml) | 725.0±239.5 | 2681.0±478.6 | 1576.0±526.9 | 0.009 |
| Plasma C5a (ng/mL) | 42.9±15.8 | 388.8±104.1 | 267.3±93.7 | 0.03 |
| C9 deposition (RawIntDen) | 0.3±0.9×10⁶ | 7.2±1.7×10⁶ | 4.3±2.2×10⁶ | 0.02 |
| Neutrophils (counts/HPF) | 2.0±1.0 | 55.0±11.0 | 37.0±16.0 | 0.03 |
| Macrophages (counts/HPF) | 2.0±1.0 | 52.0±10.0 | 33.0±18.0 | 0.03 |

### Example 8: The effect of altered dosing of sialylated sCR1(1392)-8His on renal ischemia-reperfusion injury

To assess the dose-response relationship of sCR1(1392)-8His^{SIA} in an *in vivo* model of warm renal ischemic-reperfusion (IR) injury, mice are treated with i.p. administration of either 10mg/kg, 30mg/kg or 60 mg/kg sCR1(1392)-8His^{SIA}, or vehicle control, 1 hr prior to ischemia. IR is induced as described above and IR-mediated renal damage is assessed as previously described.

The effect of maintaining elevated levels of sCR1(1392)-8His^{SIA} is assessed by administering 60 mg/kg sCR1(1392)-8His^{SIA}, or vehicle control, 1 hr prior to ischemia and 60 mg/kg sCR1(1392)-8His^{SIA}, or vehicle control 1 hour and/or 2 hours post-ischemia. IR is induced as described above and IR-mediated renal damage is assessed as previously described.

### Example 9: Generation of sCR1 variant fusions

Recombinant sCR1 fusions were generated by fusing a sCR1 variant to Human Serum Albumin (HSA) (GenBank Accession no. NP_000468), Human IgG₁ Fc (Genbank Accession No. P01857) or Human IgG₄Fc (aa99-327; GenBank Accession no. P01861) at either the Nor C-terminus of the sCR1 sequence **(Table 6)**. Recombinant fusions were made using standard cloning techniques. In the case of HSA fusions, a GS13 linker (GSGGSGGSGGSGS) was used to link the sCR1 sequence and the HSA sequence. In the case of IgG₄ Fc and IgG₁ Fc fusions, a linker was not used. For some constructs a ceruloplasmin signal peptide was employed (GenBank Accession no. NP_000087). All fusion proteins were expressed in Expi293F^{™} cells and sCR1 proteins purified as described above.

For purification of human serum albumin (HSA) tagged sCR1 fusions the culture supernatant was loaded directly onto Capture Select Human Albumin Affinity Matrix affinity resin (GE Healthcare) pre-equilibrated with 20mM Tris, pH 7.4. After all supernatant was loaded the resin was washed with 20mM Tris, pH 7.4. Resin-bounded sCR1 was block eluted with 20mM Tris, 2M MgCl2, pH 7.4, collecting eluted protein based on absorbance at 280nm. Eluted protein was loaded onto a HiLoad 26/60 superdex200 prep grade column (GE Healthcare) pre-equilibrated in mt-PBS (137mM NaCl, 27mM KCl, 8.1mM Na₂HPO₄, 1.15mM KH₂PO₄, pH 7.4) to remove any contaminating proteins and buffer exchange into desired buffer. Purified protein was concentrated using amicon ultra centrifugal filters with 50kDa MWCO to desired concentration, sterile filtered and stored at -80°C.

For purification of Fc fusion sCR1 variants the culture supernatant was loaded directly onto MabSelect SuRe affinity resin (GE Healthcare) pre-equilibrated with mt-PBS (137mM NaCl, 27mM KCl, 8.1mM Na₂HPO₄, 1.15mM KH₂PO₄, pH7.4). After all supernatant was loaded the resin was washed with mt-PBS, pH7.4. Weakly bound non-target proteins were block eluted with 0.1M Sodium Citrate, pH 5.0. Resin-bounded sCR1 was block eluted with 0.1M Sodium Citrate, pH 3.0, collecting eluted protein based on absorbance at 280nm. Eluted protein was loaded onto a HiLoad 26/60 superdex200 prep grade column (GE Healthcare) pre-equilibrated in mt-PBS to remove any contaminating proteins and buffer exchange into desired buffer. Purified protein was concentrated using amicon ultra centrifugal filters with 50kDa MWCO to desired concentration, sterile filtered and stored at -80°C.

**Table 6: sCR1 variant fusions**

| **Identifier** | **SEQ ID NO:** |
|---|---|
| sCR1(1971)-GS13-HSA SEQ ID NO: 38 | Signal peptide: SEQ ID NO: 18 |
| | sCR1 sequence: SEQ ID NO: 2 |
| | GS13 Linker: SEQ ID NO: 31 |
| | HSA sequence: SEQ ID NO: 32 |
| HSA-GS 13-sCR1(42-1971) SEQ ID NO: 39 | Signal peptide: SEQ ID NO: 36 |
| | Pro-peptide: SEQ ID NO: 42 |
| | sCR1 sequence: SEQ ID NO: 2 |
| | GS13 Linker: SEQ ID NO: 31 |
| | HSA sequence: SEQ ID NO: 32 |
| sCR1(1971)-IgG₄ Fc SEQ ID NO: 40 | Signal peptide: SEQ ID NO: 18 |
| | sCR1 sequence: SEQ ID NO: 2 |
| | IgG₄ Fc sequence: SEQ ID NO: 34 |
| IgG₄ Fc-sCR1(42-1971) SEQ ID NO: 41 | Signal peptide: SEQ ID NO: 37 |
| | sCR1 sequence: SEQ ID NO: 2 |
| | IgG₄ Fc sequence: SEQ ID NO: 34 |
| sCR1(1392)-GS13-HSA SEQ ID NO: 43 | Signal peptide: SEQ ID NO: 18 |
| | sCR1 sequence: SEQ ID NO: 3 |
| | GS13 Linker: SEQ ID NO: 31 |
| | HSA sequence: SEQ ID NO: 32 |
| HSA-GS 13-sCR1(42-1392) SEQ ID NO: 44 | Signal peptide: SEQ ID NO: 36 |
| | Pro-peptide: SEQ ID NO: 42 |
| | sCR1 sequence: SEQ ID NO: 3 |
| | GS13 Linker: SEQ ID NO: 31 |
| | HSA sequence: SEQ ID NO: 32 |
| sCR1(1392)-IgG₁ Fc SEQ ID NO: 45 | Signal peptide: SEQ ID NO: 18 |
| | sCR1 sequence: SEQ ID NO: 3 |
| | IgG₁ Fc sequence: SEQ ID NO: 33 |
| sCR1(1392)-IgG₄ Fc SEQ ID NO: 46 | Signal peptide: SEQ ID NO: 18 |
| | sCR1 sequence: SEQ ID NO: 3 |
| | IgG₄ Fc sequence: SEQ ID NO: 34 |
| IgG₄ Fc-sCR1(42-1392) SEQ ID NO: 47 | Signal peptide: SEQ ID NO: 37 |
| | sCR1 sequence: SEQ ID NO: 3 |
| | IgG₄ Fc sequence: SEQ ID NO: 34 |
| sCR1(939)-GS13-HSA SEQ ID NO: 48 | Signal peptide: SEQ ID NO: 18 |
| | sCR1 sequence: SEQ ID NO: 4 |
| | GS13 Linker: SEQ ID NO: 31 |
| | HSA sequence: SEQ ID NO: 32 |
| sCR1(939)-IgG₄ Fc SEQ ID NO: 49 | Signal peptide: SEQ ID NO: 18 |
| | sCR1 sequence: SEQ ID NO: 3 |
| | IgG₄ Fc sequence: SEQ ID NO: 34 |
| IgG₄ Fc-sCR1(42-939) SEQ ID NO: 50 | Signal peptide: SEQ ID NO: 37 |
| | sCR1 sequence: SEQ ID NO: 3 |
| | IgG₄ Fc sequence: SEQ ID NO: 34 |

All sCR1 variants with N- or C- terminal conjugation to HSA, IgG₁ Fc or IgG₄ Fc had complement inhibitory activity in the classical, lectin and alternative pathways, as measured using the Wieslab assay, as previously described.

As shown in **Tables 7 and 8** below, sCR1(1392)-GS13-HSA and sCR1(1392)-IgG₄ Fc had increased complement inhibitory activity compared to sCR1(1392)-8His as measured in all three complement pathways (i.e., classical, lectin and alternative) in the Wieslab assay as well as in the hemolysis (i.e., CH50 and ApH50) inhibition assays. sCR1(1392)-IgG₄ Fc fusions had about a 2-fold increase in complement inhibitory activity in the classical pathway (mean 2.31±0.16) and in the lectin pathway (mean 2.37±0.44) and an 7-8-fold increase in complement inhibitory activity in the alternative pathway (mean 7.61±2.52) compared to sCR1(1392)-8His. C-terminal fusion with HSA and IgG₄ Fc did not adversely affect complement inhibitory activity of sCR1(1392).

**Table 7: Relative in vitro activity of sCR1 variant fusions in Wieslab assays**

| | **Experiment #** | | | | | | | |
|---|---|---|---|---|---|---|---|---|
| **sCR1 fusion** | **#1** | **#2** | **#3** | **#4** | **#5** | **#6** | **#7** | **Mean ± SEM** |
| | **Classical (IC₅₀) (pM)** | | | | | | | |
| sCR1(1392)-8His | 402 | 215 | 720 | 678 | 710 | 779 | 645 | 592.7 ± 77.7 |
| sCR1(1392)-GS13-HSA | 490 | 294 | 693 | 787 | 977 | - | 710 | 658.5 ± 97.1 |
| HSA-GS 13-sCR1(1392) | - | - | 1030 | 127 0 | 172 9 | - | - | 1343 ± 205.1 |
| sCR1(1392)-IgG₄Fc | 207 | 68 | 302 | 368 | 311 | 318 | 303 | 268.1 ± 37.9 |
| sIgG₄Fc-sCR1(1392) | - | - | 588 | 707 | 907 | - | - | 734 ± 93.1 |

| | **Lectin (IC₅₀) (pM)** | | | | | | | |
|---|---|---|---|---|---|---|---|---|
| sCR1 (1392)-8His | 428 | 464 | 704 | 468 | 604 | 549 | 532 | 535.6 ± 36.0 |
| sCR1(1392)-GS13-HSA | 391 | 533 | 678 | 509 | 946 | - | 563 | 604.3 ± 77.7 |
| HSA-GS 13-sCR1(1392) | - | - | 983 | 897 | 139 8 | - | | 1092.7 ± 154.7 |
| sCR1(1392)-IgG₄Fc | 89 | 163 | 300 | 303 | 355 | 321 | 314 | 263.6 ± 37.1 |
| sIgG₄Fc-sCR1(1392) | - | - | 579 | 565 | 976 | - | - | 706.7 ± 134.7 |

| | **Alternative (IC₅₀) (pM)** | | | | | | | |
|---|---|---|---|---|---|---|---|---|
| sCR1(1392)-8His | 384 | 520 | 266 | 572 | 342 | 584 | 137 | 400.7 ± 63.4 |
| sCR1(1392)-GS13-HSA | 326 | 351 | 248 | 449 | 304 | - | - | 235.5 ± 73.3 |
| HSA-GS 13-sCR1(1392) | - | - | 616 | 731 | 402 | - | - | 583 ± 96.4 |
| sCR1(1392)-IgG₄Fc | 65 | 28 | 114 | 162 | 31 | 136 | - | 78 ± 22.5 |
| IgG₄Fc-sCR1(1392) | - | - | 139 | 480 | 240 | - | - | 286.3 ± 101.1 |

**Table 8: Relative in vitro activity of sCR1 variant fusions in hemolysis assays**

| **sCR1 fusion** | **Exp't No** | **Classical (IC₅₀) (pM)** |
|---|---|---|
| sCR1(1392)-8His | 1 | 335 |
| | 2 | 376 |
| sCR1(1392) + HSA | 1 | 437 |
| sCR1(1392)-GS13-HSA | 1 | 245 |
| HSA alone | 1 | No activity |
| sCR1(1392)-8His + IgG₄Fc | 2 | 343 |
| sCR1(1392)-IgG₄Fc | 2 | 251 |
| sIgG₄Fc alone | 2 | No activity |

| | | **Alternative (IC₅₀) (pM)** |
|---|---|---|
| sCR1(1392)-8His | 3 | 1240 |
| | 4 | 755 |
| sCR1(1392) + HSA | 3 | 858 |
| sCR1(1392)-GS13-HSA | 3 | 1012 |
| HSA alone | 3 | No activity |
| sCR1(1392)-8His + IgG₄Fc | 4 | 657 |
| sCR1(1392)-IgG₄Fc | 4 | 658 |
| sIgG₄Fc alone | 4 | No activity |

A recombinant sCR1-HSA fusion (sCR1(1392)-HSA; SEQ ID NO: 51) was generated as described above, expressed in CHO Xceed^{®} cells and purified as described above. Complement activity in the classical and lectin pathways was measured using the Wieslab assay confirming that the CHO Xceed^{®}-derived material had similar activity compared to Expi293F^{™}-derived material.

### Example 10: Dimeric Fc sCR1 variant fusions have increased inhibitory activity compared to monomeric Fc sCR1 variant fusions

Recombinant sCR1 fusions were generated as previously described with C-terminal conjugation to a dimeric IgG₁ Fc, a dimeric IgG₄ Fc or a monomeric IgG₄ Fc.

As shown below in **Tables 9 and 10,** sCR1(1392)-IgG₁ Fc and sCR1(1392)-IgG₄ Fc had increased complement inhibitory activity compared to sCR1(1392)-8His as measured in all three complement pathways (i.e., classical, lectin and alternative) in the Wieslab assay as well as in the hemolysis (i.e., CH50 and ApH50) inhibition assays. In particular, sCR1(1392)-dimeric IgG₁ Fc fusions and sCR1(1392)-dimeric IgG₄ Fc fusions had about a 2-fold increase in complement inhibitory activity in the classical and lectin pathways and an 4-fold increase in complement inhibitory activity in the alternative pathway compared to sCR1(1392)-8His and sCR1(1392)- monomeric IgG₄ Fc fusions. These results also show that N-terminal fusions adversely impact the complement inhibitory activity of sCR1(1392) in the alternative pathway.

**Table 9: Relative in vitro activity of sCR1 variant fusions in Wieslab assays**

| **sCR1 fusion** | **Classical (IC₅₀) (pM)** | **Lectin (IC₅₀) (pM)** | **Alternative (IC₅₀) (pM)** |
|---|---|---|---|
| sCR1(1392)-8His | 778.9 | 549.3 | 583.6 |
| sCR1(1392)-IgG₄Fc | 318.5 | 321.1 | 136.6 |
| sCR1(1392)-IgG₁Fc | 241.9 | 266.4 | 150.0 |
| sCR1(1392)-monomericIgG₄Fc | 778.9 | 549.3 | 569.0 |

**Table 10: Relative in vitro activity of sCR1 variant fusions in hemolysis assays**

| **sCR1 fusion** | **Classical (IC₅₀) (pM)** | **Alternative (IC₅₀) (pM)** |
|---|---|---|
| sCR1(1392)-8His | 427 | 956 |
| sCR1(1392)-IgG₄Fc | 151 | 165 |
| IgG₄Fc-sCR1(1392) | 178 | 2061 |
| sCR1(1392)-monomericIgG₄Fc | 259 | 1060 |

## Claims

1. A soluble complement receptor type 1 (sCR1) variant for use in a method of inhibiting complement activity in a subject, the method comprising administering a soluble complement receptor type 1 (sCR1) variant to the subject, the sCR1 variant comprising an amino acid sequence corresponding to amino acids 42 to 1392 of SEQ ID NO: 1,
wherein the sCR1 variant comprises long homologous repeat (LHR) regions LHR-A, LHR-B and LHR-C, but is lacking LHR-D.

2. The sCR1 variant for use according to claim 1, wherein the sCR1 variant has increased complement inhibitory activity compared to a sCR1 comprising a sequence set forth in SEQ ID NO: 2.

3. The sCR1 variant for use according to any one of claims 1 or 2, wherein the sCR1 variant has increased complement inhibitory activity in the classical pathway, the lectin pathway and/or alternative complement pathway compared to a sCR1 comprising a sequence set forth in SEQ ID NO: 2.

4. The sCR1 variant for use according to any one of claims 1 to 3, wherein the sCR1 variant is conjugated to a half-life extending moiety or a further soluble complement inhibitor.

5. The sCR1 variant for use of claim 4, wherein the half-life extending moiety is selected from the group consisting of a human serum albumin or functional fragment thereof, a monomeric or dimeric immunoglobulin Fc region or functional fragment thereof, afamin, alpha-fetoprotein, vitamin D binding protein, antibody fragments that bind to albumin and polymers.

6. The sCR1 variant for use of claim 4, wherein the further soluble complement inhibitor is selected from the group consisting of C1-inhibitor (C1-INH), Factor I, (fl), Factor H (fH), complement Factor H related protein (CFHR), C4b-binding protein (C4bp), soluble CD55 (decay accelerating factor (DAF)), soluble CD46 (membrane cofactor protein (MCP)), soluble CD59 (protectin), soluble complement receptor 2 (sCR2), TT30 (CR2-fH) and Cobra venom factor (CVF).

7. The sCR1 variant for use according to any one of claims 1 to 6, wherein the subject is suffering from, or at risk of, a complement mediated disorder.

8. The sCR1 variant for use of claim 7, wherein the complement mediated disorder is selected from the group consisting of transplant rejection (including delayed graft function, graft salvage and antibody mediated rejection), solid organ transplantation, a nephropathy, ischemia-reperfusion injury, neuromyelitis optica, myasthenia gravis, a glomerular pathology, lupus nephritis (acute and chronic), IgA nephropathy, bullous pemphigoid, anti-phospholipid syndrome, uveitis, a neurological disorder, Parkinson's disease, Huntington's disease, cerebral infarction, motor neuron disease, autoimmune haemolytic anemia, ANCA-associated vasculitis, chronic inflammatory demyelinating polyneuropathy, ischemic stroke (with and without reperfusion), traumatic brain injury, somatic trauma and anti-glomerular basement membrane (GBM) nephritis.

9. A soluble complement receptor type 1 (sCR1) conjugate comprising:
(i) an sCR1 variant comprising an amino acid sequence corresponding to amino acids 42 to 1392 of SEQ ID NO: 1; and
(ii) a compound selected from the group consisting of:
a) a half-life extending moiety; and
b) a further soluble complement inhibitor,
wherein the sCR1 variant comprises long homologous repeat (LHR) regions LHR-A, LHR-B and LHR-C, but is lacking LHR-D.

10. The conjugate of claim 9, wherein the half-life extending moiety is selected from the group consisting of a human serum albumin or functional fragment thereof, an immunoglobulin Fc region or functional fragment thereof, afamin, alpha-fetoprotein, vitamin D binding protein, antibody fragments that bind to albumin and polymers.

11. The conjugate of claim 9 or 10, wherein the further soluble complement inhibitor is selected from the group consisting of C1-inhibitor (C1-INH), Factor I (fl), Factor H (fH), complement Factor H related protein (CFHR), C4b-binding protein (C4bp), soluble CD55 (decay accelerating factor (DAF)), soluble CD46 (membrane cofactor protein (MCP)), soluble CD59 (protectin), soluble complement receptor 2 (sCR2), TT30 (CR2-fH) and Cobra venom factor (CVF).

12. The sCR1 conjugate according to any one of claims 9 to 11, wherein the sCR1 conjugate has increased complement inhibitory activity compared to a sCR1 comprising a sequence set forth in SEQ ID NO: 2.

13. The sCR1 conjugate according to any one of claims 9 to 12, wherein the sCR1 variant has increased complement inhibitory activity in the classical pathway, the lectin pathway and/or alternative complement pathway compared to a sCR1 comprising a sequence set forth in SEQ ID NO: 2.

14. A composition comprising the sCR1 conjugate according to any one of claims 9 to 13, and a pharmaceutical carrier and/or excipient.

15. The composition of claim 14, wherein at least 30% of the sCR1 variant glycoforms in the composition comprise sialylated glycans.

16. A composition comprising an sCR1 variant, wherein at least 30% of the sCR1 variant glycoforms in the composition comprise sialylated glycans, and wherein sCR1 variant comprises an amino acid sequence an amino acid sequence corresponding to amino acids 42 to 1392 of SEQ ID NO: 1,
wherein the sCR1 variant comprises long homologous repeat (LHR) regions LHR-A, LHR-B and LHR-C, but is lacking LHR-D.

17. A sCR1 conjugate according to any one of claims 9 to 13, or a composition of any one of claims 14 to 16, for use in treating or preventing a disease or condition in a subject.

18. The sCR1 conjugate or composition for use according to claim 17, wherein the disease or condition is a complement mediated disorder.

19. The sCR1 conjugate or composition for use according to claim 17 or 18, wherein the subject is suffering from, or at risk of, a complement mediated disorder or condition.

20. The sCR1 conjugate or composition for use according to any one of claims 17 to 19, wherein the complement mediated disorder is selected from the group consisting of transplant rejection (including delayed graft function, graft salvage and antibody mediated rejection), solid organ transplantation, a nephropathy, ischemia-reperfusion injury, neuromyelitis optica, myasthenia gravis, a glomerular pathology, lupus nephritis (acute and chronic), IgA nephropathy, bullous pemphigoid, anti-phospholipid syndrome, uveitis, a neurological disorder, Parkinson's disease, Huntington's disease, cerebral infarction, motor neuron disease, autoimmune haemolytic anemia, ANCA-associated vasculitis, chronic inflammatory demyelinating polyneuropathy, ischemic stroke (with and without reperfusion), traumatic brain injury, somatic trauma and anti-glomerular basement membrane (GBM) nephritis.

21. A kit for use in treating or preventing a complement mediated disorder in a subject, the kit comprising:
(a) at least one sCR1 conjugate according to any one of claims 9 to 13, or composition of any one of claims 14 to 16;
(b) instructions for using the kit in treating or preventing the complement mediated disorder in the subject; and
(c) optionally, at least one further therapeutically active compound or drug.

## Patentansprüche

1. Variante des löslichen Komplementrezeptors Typ 1 (sCR1) zur Verwendung bei einem Verfahren zur Hemmung der Komplementaktivität in einem Individuum, wobei das Verfahren das Verabreichen einer Variante des löslichen Komplementrezeptors Typ 1 (sCR1) an das Individuum umfasst, wobei die sCR1-Variante eine Aminosäuresequenz umfasst, die den Aminosäuren 42 bis 1392 von SEQ ID NO: 1 entspricht,
wobei die sCR1-Variante die langen homologen Repeat(LHR)-Regionen LHR-A, LHR-B und LHR-C umfasst, ihr jedoch LHR-D fehlt.

2. sCR1-Variante zur Verwendung nach Anspruch 1, wobei die sCR1-Variante erhöhte Komplement-inhibitorische Aktivität im Vergleich zu einem sCR1, der eine in SEQ ID NO: 2 dargestellte Sequenz umfasst, aufweist.

3. sCR1-Variante zur Verwendung nach einem der Ansprüche 1 oder 2, wobei die sCR1-Variante erhöhte Komplement-inhibitorische Aktivität im klassischen Weg, im Lektinweg und/oder im alternativen Komplementweg im Vergleich zu einem sCR1, der eine in SEQ ID NO: 2 dargestellte Sequenz umfasst, aufweist.

4. sCR1-Variante zur Verwendung nach einem der Ansprüche 1 bis 3, wobei die sCR1-Variante an eine die Halbwertszeit verlängernde Einheit oder einen weiteren löslichen Komplementinhibitor konjugiert ist.

5. sCR1-Variante zur Verwendung nach Anspruch 4, wobei die die Halbwertszeit verlängernde Einheit aus der Gruppe ausgewählt ist, die aus einem menschlichen Serumalbumin oder einem funktionellen Fragment davon, einer monomeren oder dimeren Immunglobulin-Fc-Region oder einem funktionellen Fragment davon, Afamin, alpha-Fetoprotein, Vitamin D-bindendem Protein, Antikörperfragmenten, die an Albumin binden, und Polymeren besteht.

6. sCR1-Variante zur Verwendung nach Anspruch 4, wobei der weitere lösliche Komplementinhibitor aus der Gruppe ausgewählt ist, die aus C1-Inhibitor (C1-INH), Faktor I (fl), Faktor H (fH), Komplementfaktor-H-verwandtem Protein (CFHR), C4b-bindendem Protein (C4bp), löslichem CD55 (Zerfallsbeschleunigungsfaktor (decay accelerating factor, DAF)), löslichem CD46 (Membran-Cofaktor-Protein (MCP)), löslichem CD59 (Protectin), löslichem Komplementrezeptor 2 (sCR2), TT30 (CR2-fH) und Kobragiftfaktor (CVF) besteht.

7. sCR1-Variante zur Verwendung nach einem der Ansprüche 1 bis 6, wobei das Individuum an einer Komplement-vermittelten Störung leidet oder einem Risiko für eine solche ausgesetzt ist.

8. sCR1-Variante zur Verwendung nach Anspruch 7, wobei die Komplement-vermittelte Störung aus der Gruppe ausgewählt ist, die aus Transplantatabstoßung (einschließlich verzögerter Transplantatfunktion, Transplantaterhaltung und Antikörper-vermittelter Abstoßung), Transplantation solider Organe, einer Nephropathie, Ischämie-Reperfusions-Verletzung, Neuromyelitis optica, Myasthenia gravis, einer glomerulären Pathologie, (akuter und chronischer) Lupus-Nephritis, IgA-Nephropathie, bullösem Pemphigoid, Anti-Phospholipid-Syndrom, Uveitis, einer neurologischen Störung, Parkinson-Krankheit, Huntington-Krankheit, Hirninfarkt, Motoneuron-Krankheit, autoimmuner hämolytischer Anämie, ANCA-assoziierter Vaskulitis, chronisch entzündlicher demyelinisierender Polyneuropathie, ischämischem Schlaganfall (mit und ohne Reperfusion), traumatischer Hirnverletzung, somatischem Trauma und Anti-glomeruläre-Basalmembran(GBM)-Nephritis besteht.

9. Löslicher-Komplementrezeptor-Typ 1(sCR1)-Konjugat, umfassend:
(i) eine sCR1-Variante, die eine Aminosäuresequenz umfasst, die den Aminosäuren 42 bis 1392 von SEQ ID NO: 1 entspricht, und
(ii) eine Verbindung, die aus der Gruppe ausgewählt ist, die aus:
a) einer die Halbwertszeit verlängernden Einheit und
b) einem weiteren löslichen Komplementinhibitor besteht,
wobei die sCR1-Variante die langen homologen Repeat(LHR)-Regionen LHR-A, LHR-B und LHR-C umfasst, ihr jedoch LHR-D fehlt.

10. Konjugat nach Anspruch 9, wobei die die Halbwertszeit verlängernde Einheit aus der Gruppe ausgewählt ist, die aus einem menschlichen Serumalbumin oder einem funktionellen Fragment davon, einer Immunglobulin-Fc-Region oder einem funktionellen Fragment davon, Afamin, alpha-Fetoprotein, Vitamin D-bindendem Protein, Antikörperfragmenten, die an Albumin binden, und Polymeren besteht.

11. Konjugat nach Anspruch 9 oder 10, wobei der weitere lösliche Komplementinhibitor aus der Gruppe ausgewählt ist, die aus C1-Inhibitor (C1-INH), Faktor I (fl), Faktor H (fH), Komplementfaktor-H-verwandtem Protein (CFHR), C4b-bindendem Protein (C4bp), löslichem CD55 (Zerfallsbeschleunigungsfaktor (decay accelerating factor, DAF)), löslichem CD46 (Membran-Cofaktor-Protein (MCP)), löslichem CD59 (Protectin), löslichem Komplementrezeptor 2 (sCR2), TT30 (CR2-fH) und Kobragiftfaktor (CVF) besteht.

12. sCR1-Konjugat nach einem der Ansprüche 9 bis 11, wobei das sCR1-Konjugat erhöhte Komplement-inhibitorische Aktivität im Vergleich zu einem sCR1, der eine in SEQ ID NO: 2 dargestellte Sequenz umfasst, aufweist.

13. sCR1-Konjugat nach einem der Ansprüche 9 bis 12, wobei die sCR1-Variante erhöhte Komplement-inhibitorische Aktivität im klassischen Weg, im Lektinweg und/oder im alternativen Komplementweg im Vergleich zu einem sCR1, der eine in SEQ ID NO: 2 dargestellte Sequenz umfasst, aufweist.

14. Zusammensetzung, umfassend das sCR1-Konjugat nach einem der Ansprüche 9 bis 13 und einen pharmazeutischen Träger und/oder Exzipienten.

15. Zusammensetzung nach Anspruch 14, wobei mindestens 30 % der Glykoformen der sCR1-Variante in der Zusammensetzung sialylierte Glykane umfassen.

16. Zusammensetzung, umfassend eine sCR1-Variante, wobei mindestens 30 % der Glykoformen der sCR1-Variante in der Zusammensetzung sialylierte Glykane umfassen und wobei die sCR1-Variante eine Aminosäuresequenz umfasst, die den Aminosäuren 42 bis 1392 von SEQ ID NO: 1 entspricht,
wobei die sCR1-Variante die langen homologen Repeat(LHR)-Regionen LHR-A, LHR-B und LHR-C umfasst, ihr jedoch LHR-D fehlt.

17. sCR1-Konjugat nach einem der Ansprüche 9 bis 13 oder Zusammensetzung nach einem der Ansprüche 14 bis 16 zur Verwendung bei der Behandlung oder Vorbeugung einer Krankheit oder eines Zustands in einem Individuum.

18. sCR1-Konjugat oder Zusammensetzung zur Verwendung nach Anspruch 17, wobei die Krankheit oder der Zustand eine Komplement-vermittelte Störung ist.

19. sCR1-Konjugat oder Zusammensetzung zur Verwendung nach Anspruch 17 oder 18, wobei das Individuum an einer Komplement-vermittelten Störung oder einem Komplement-vermittelten Zustand leidet oder einem Risiko für eine solche bzw. einen solchen ausgesetzt ist.

20. sCR1-Konjugat oder Zusammensetzung zur Verwendung nach einem der Ansprüche 17 bis 19, wobei die Komplement-vermittelte Störung aus der Gruppe ausgewählt ist, die aus Transplantatabstoßung (einschließlich verzögerter Transplantatfunktion, Transplantaterhaltung und Antikörper-vermittelter Abstoßung), Transplantation solider Organe, einer Nephropathie, Ischämie-Reperfusions-Verletzung, Neuromyelitis optica, Myasthenia gravis, einer glomerulären Pathologie, (akuter und chronischer) Lupus-Nephritis, IgA-Nephropathie, bullösem Pemphigoid, Anti-Phospholipid-Syndrom, Uveitis, einer neurologischen Störung, Parkinson-Krankheit, Huntington-Krankheit, Hirninfarkt, Motoneuron-Krankheit, autoimmuner hämolytischer Anämie, ANCA-assoziierter Vaskulitis, chronisch entzündlicher demyelinisierender Polyneuropathie, ischämischem Schlaganfall (mit und ohne Reperfusion), traumatischer Hirnverletzung, somatischem Trauma und Anti-glomeruläre-Basalmembran(GBM)-Nephritis besteht.

21. Kit zur Verwendung bei der Behandlung oder Vorbeugung einer Komplement-vermittelten Störung in einem Individuum, wobei das Kit Folgendes umfasst:
(a) mindestens ein sCR1-Konjugat nach einem der Ansprüche 9 bis 13 oder eine Zusammensetzung nach einem der Ansprüche 14 bis 16,
(b) Anleitungen für die Verwendung des Kits bei der Behandlung oder Vorbeugung der Komplement-vermittelten Störung in dem Individuum und
(c) optional mindestens eine weitere therapeutisch wirksame Verbindung oder einen weiteren Arzneistoff.

## Revendications

1. Variant du récepteur de complément soluble de type 1 (sCR1) pour une utilisation dans un procédé d'inhibition de l'activité du complément chez un sujet, le procédé comprenant une administration d'un variant du récepteur de complément soluble de type 1 (sCR1) au sujet, le variant de sCR1 comprenant une séquence d'acides aminés correspondant aux acides aminés 42 à 1392 de la SEQ ID NO: 1,
le variant de sCR1 comprenant des régions de répétition homologue longue (LHR) LHR-A, LHR-B et LHR-C, mais étant dépourvu de LHR-D.

2. Variant de sCR1 pour une utilisation selon la revendication 1, le variant de sCR1 ayant une activité inhibitrice du complément augmentée par comparaison avec un sCR1 comprenant une séquence indiquée dans la SEQ ID NO: 2.

3. Variant de sCR1 pour une utilisation selon l'une quelconque des revendications 1 ou 2, le variant de sCR1 ayant une activité inhibitrice du complément augmentée dans la voie classique, la voie de la lectine et/ou une voie du complément alternative par comparaison avec un sCR1 comprenant une séquence indiquée dans la SEQ ID NO: 2.

4. Variant de sCR1 pour une utilisation selon l'une quelconque des revendications 1 à 3, le variant de sCR1 étant conjugué à une entité étendant la demi-vie ou à un autre inhibiteur du complément soluble.

5. Variant de sCR1 pour une utilisation selon la revendication 4, l'entité étendant la demi-vie étant choisie dans le groupe constitué par une albumine sérique humaine ou un fragment fonctionnel correspondant, une région Fc d'immunoglobuline monomérique ou dimérique ou un fragment fonctionnel correspondant, une afamine, une alpha-fétoprotéine, une protéine de liaison à la vitamine D, des fragments d'anticorps qui se lient à une albumine et des polymères.

6. Variant de sCR1 pour une utilisation selon la revendication 4, l'autre inhibiteur du complément soluble étant choisi dans le groupe constitué par l'inhibiteur du C1 (C1-INH), le facteur I (fI), le facteur H (fH), la protéine apparentée au facteur H du complément (CFHR), la protéine de liaison au C4b (C4bp), la CD55 soluble (facteur d'accélération de la décomposition (DAF)), la CD46 soluble (protéine cofacteur membranaire (MCP)), la CD59 soluble (protectine), le récepteur du complément soluble 2 (sCR2), la TT30 (CR2-fH) et le facteur du venin de Cobra (CVF).

7. Variant de sCR1 pour une utilisation selon l'une quelconque des revendications 1 à 6, le sujet souffrant d'un, ou étant à risque d'un, trouble médié par le complément.

8. Variant de sCR1 pour une utilisation selon la revendication 7, le trouble médié par le complément étant choisi dans le groupe constitué par un rejet de greffe (y compris le retard de fonction du greffon, le sauvetage du greffon et le rejet médié par les anticorps), une transplantation d'organe solide, une néphropathie, une lésion d'ischémie-reperfusion, une neuromyélite optique, une myasthénie grave, une pathologie glomérulaire, une néphrite lupique (aiguë et chronique), une néphropathie à IgA, une pemphigoïde bulleuse, un syndrome des anti-phospholipides, une uvéite, un trouble neurologique, la maladie de Parkinson, la maladie de Huntington, un infarctus cérébral, une maladie du motoneurone, une anémie hémolytique auto-immune, une vascularite associée à l'ANCA, une polyneuropathie inflammatoire démyélinisante chronique, un accident vasculaire cérébral ischémique (avec ou sans reperfusion), une lésion cérébrale traumatique, un traumatisme somatique et une néphrite anti-membrane basale glomérulaire (GBM).

9. Conjugué du récepteur de complément soluble de type 1 (sCR1) comprenant :
(i) un variant de sCR1 comprenant une séquence d'acides aminés correspondant aux acides aminés 42 à 1392 de la SEQ ID NO: 1 ; et
(ii) un composé choisi dans le groupe constitué par :
a) une entité étendant la demi-vie ; et
b) un autre inhibiteur du complément soluble,
le variant de sCR1 comprenant des régions de répétition homologue longue (LHR) LHR-A, LHR-B et LHR-C, mais étant dépourvu de LHR-D.

10. Conjugué selon la revendication 9, l'entité étendant la demi-vie étant choisie dans le groupe constitué par une albumine sérique humaine ou un fragment fonctionnel correspondant, une région Fc d'immunoglobuline ou un fragment fonctionnel correspondant, une afamine, une alpha-fétoprotéine, une protéine de liaison à la vitamine D, des fragments d'anticorps qui se lient à une albumine et des polymères.

11. Conjugué selon la revendication 9 ou 10, l'autre inhibiteur du complément soluble étant choisi dans le groupe constitué par l'inhibiteur du C1 (C1-INH), le facteur I (fI), le facteur H (fH), la protéine apparentée au facteur H du complément (CFHR), la protéine de liaison au C4b (C4bp), la CD55 soluble (facteur d'accélération de la décomposition (DAF)), la CD46 soluble (protéine cofacteur membranaire (MCP)), la CD59 soluble (protectine), le récepteur du complément soluble 2 (sCR2), la TT30 (CR2-fH) et le facteur du venin de Cobra (CVF).

12. Conjugué de sCR1 selon l'une quelconque des revendications 9 à 11, le conjugué de sCR1 ayant une activité inhibitrice du complément augmentée par comparaison avec un sCR1 comprenant une séquence indiquée dans la SEQ ID NO: 2.

13. Conjugué de sCR1 selon l'une quelconque des revendications 9 à 12, le variant de sCR1 ayant une activité inhibitrice du complément augmentée dans la voie classique, la voie de la lectine et/ou une voie du complément alternative par comparaison avec un sCR1 comprenant une séquence indiquée dans la SEQ ID NO: 2.

14. Composition comprenant le conjugué de sCR1 selon l'une quelconque des revendications 9 à 13, et un support et/ou excipient pharmaceutique.

15. Composition selon la revendication 14, au moins 30 % des glycoformes de variant de sCR1 dans la composition comprenant des glycanes sialylés.

16. Composition comprenant un variant de sCR1, au moins 30 % des glycoformes de variant de sCR1 dans la composition comprenant des glycanes sialylés, et un variant de sCR1 comprenant une séquence d'acides aminés correspondant aux acides aminés 42 à 1392 de la SEQ ID NO: 1,
le variant de sCR1 comprenant des régions de répétition homologue longue (LHR) LHR-A, LHR-B et LHR-C, mais étant dépourvu de LHR-D.

17. Conjugué de sCR1 selon l'une quelconque des revendications 9 à 13, ou composition selon l'une quelconque des revendications 14 à 16, pour une utilisation dans le traitement ou la prévention d'une maladie ou d'une affection chez un sujet.

18. Conjugué ou composition de sCR1 pour une utilisation selon la revendication 17, la maladie ou l'affection étant un trouble médié par le complément.

19. Conjugué ou composition de sCR1 pour une utilisation selon la revendication 17 ou 18, le sujet souffrant d'un(e), ou étant à risque d'un(e), trouble ou affection médié(e) par le complément.

20. Conjugué ou composition de sCR1 pour une utilisation selon l'une quelconque des revendications 17 à 19, le trouble médié par le complément étant choisi dans le groupe constitué par un rejet de greffe (y compris le retard de fonction du greffon, le sauvetage du greffon et le rejet médié par les anticorps), une transplantation d'organe solide, une néphropathie, une lésion d'ischémie-reperfusion, une neuromyélite optique, une myasthénie grave, une pathologie glomérulaire, une néphrite lupique (aiguë et chronique), une néphropathie à IgA, une pemphigoïde bulleuse, un syndrome des anti-phospholipides, une uvéite, un trouble neurologique, la maladie de Parkinson, la maladie de Huntington, un infarctus cérébral, une maladie du motoneurone, une anémie hémolytique auto-immune, une vascularite associée à l'ANCA, une polyneuropathie inflammatoire démyélinisante chronique, un accident vasculaire cérébral ischémique (avec ou sans reperfusion), une lésion cérébrale traumatique, un traumatisme somatique et une néphrite anti-membrane basale glomérulaire (GBM).

21. Kit pour une utilisation dans le traitement ou la prévention d'un trouble médié par le complément chez un sujet, le kit comprenant :
(a) au moins un conjugué de sCR1 selon l'une quelconque des revendications 9 à 13, ou une composition selon l'une quelconque des revendications 14 à 16 ;
(b) des instructions pour une utilisation du kit dans le traitement ou la prévention du trouble médié par le complément chez le sujet ; et
(c) éventuellement, au moins un autre composé ou médicament thérapeutiquement actif.
